(19) Europäisches Patentamt
European Patent Office
Office européen des brevets

(11) **EP 2 582 650 B1**

(12) **EUROPÄISCHE PATENTSCHRIFT**

(45) Veröffentlichungstag und Bekanntmachung des Hinweises auf die Patenterteilung:
**12.07.2017 Patentblatt 2017/28**

(21) Anmeldenummer: **11720723.3**

(22) Anmeldetag: **05.05.2011**

(51) Int Cl.:
*C07C 29/32* (2006.01)    *C07C 31/12* (2006.01)
*C07C 45/29* (2006.01)    *C07C 47/02* (2006.01)
*C07C 47/06* (2006.01)

(86) Internationale Anmeldenummer:
**PCT/EP2011/002233**

(87) Internationale Veröffentlichungsnummer:
**WO 2011/157322 (22.12.2011 Gazette 2011/51)**

(54) **KATALYTISCHE KONVERSION VON ALKOHOLEN UND ALDEHYDEN**

CATALYTIC CONVERSION OF ALCOHOLS AND ALDEHYDES

CONVERSION CATALYTIQUE D'ALCOOLS ET D'ALDÉHYDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.06.2010 DE 102010024099**

(43) Veröffentlichungstag der Anmeldung:
**24.04.2013 Patentblatt 2013/17**

(73) Patentinhaber: **Fraunhofer-Gesellschaft zur Förderung der angewandten Forschung e.V.
80686 München (DE)**

(72) Erfinder:
• **MENNE, Andreas
45470 Mülheim an der Ruhr (DE)**
• **KRAFT, Axel
45739 Oer-Erkenschwick (DE)**

(56) Entgegenhaltungen:
**EP-A1- 0 253 540    EP-A1- 1 052 234
EP-A2- 0 311 297    US-A- 4 825 013**

• **OLSON ET AL: "Higher- Alcohols Biorefinery-Improvement of Catalyst for Ethanol Conversion", APPLIED BIOCHEMISTRY AND BIOTECHNOLOGY, THE HUMANA PRESS, INC, US, Bd. 113-116, 1. Januar 2004 (2004-01-01), Seiten 913-932, XP009152756, ISSN: 0273-2289**

**Beschreibung**

Die vorliegende Erfindung betrifft das technische Gebiet der katalytischen Umsetzung bzw. Konversion von Alkoholen und Aldehyden, insbesondere zur Herstellung höherer Alkohole und/oder Aldehyde oder deren Gemischen.

Insbesondere betrifft die vorliegende Erfindung ein Verfahren zur Herstellung höherer Alkohole und/oder Aldehyde durch katalytische Umsetzung von Ethanol.

Weiterhin betrifft die vorliegende Erfindung die Verwendung eines mit mindestens einem Metall versehenen Aktivkohlesubstrats als Katalysator für die katalytische Umsetzung von Ethanol.

Darüber hinaus betrifft die vorliegende Erfindung ein Verfahren zur Kettenverlängerung von Oxo- und/oder Hydroxyfunktionen aufweisenden Kohlenstoffverbindungen durch katalytische Umsetzung.

Schließlich betrifft die vorliegende Erfindung die Verwendung eines mit mindestens einem Metall versehenen Aktivkohlesubstrats als Katalysator für die katalytische Kettenverlängerung von Oxo- und/oder Hydroxyfunktionen aufweisenden Kohlenstoffverbindungen.

Höhere bzw. höhermolekulare Alkohole und Aldehyde sowie deren Gemische, insbesondere $C_3$-$C_{30}$-Verbindungen (d. h. Verbindungen mit 3 bis 30 Kohlenstoffatomen) des vorgenannten Typs, finden zahlreiche Verwendungen auf unterschiedlichsten technischen Gebieten und sind daher von großer technischer Bedeutung: So werden höhere Alkohole und Aldehyde in technischen Prozessen beispielsweise als Lösemittel, als Additive für Kunststoffe, Farben und Lacke sowie als Treibstoffe bzw. Treibstoffzusätze oder aber als Edukte bzw. Bausteine für weiterführende Synthesen eingesetzt.

Unter den Begriffen "höherer Alkohol" und "höherer Aldehyd" werden im Rahmen der vorliegenden Erfindung insbesondere organische Verbindungen mit mindestens einer Hydroxy- und/oder Aldehydfunktion und einer mindestens drei Atome umfassenden Kohlenstoffkette, insbesondere $C_3$-$C_{30}$-Verbindungen, verstanden. Die Kohlenstoffkette kann dabei linear oder verzweigt sein und kann gegebenenfalls durch Etherfunktionen unterbrochen sein.

Weiterhin handelt es sich bei den höheren Alkoholen und/oder Aldehyden im Allgemeinen um Verbindungen, welche von aliphatischen Kohlenwasserstoffen abgeleitet sind, wobei es jedoch auch vorgesehen sein kann, dass ein Teil der Wasserstoffatome z. B. gegen funktionelle Gruppen oder Heteroatome, beispielsweise Halogenatome, ausgetauscht ist. Gleichfalls ist es jedoch auch möglich, dass es sich bei den höheren Alkoholen und/oder Aldehyden um aromatische oder teilweise aromatische Systeme mit mindestens einer Hydroxy- und/oder Aldehydfunktion handelt.

Insbesondere die $C_3$-$C_{10}$-Alkohole bzw. -Aldehyde sind von herausragender technischer Bedeutung: Die primären Alkohole dieses Typs werden als Lösemittel oder zur Herstellung von Weichmachern und Tensiden sowie als Additive in Lacken und Farben eingesetzt.

Darüber hinaus lassen sich die Verbindungen auch als Edukte bzw. Bausteine für weitere großtechnische Verfahren nutzen. Von besonderer Bedeutung ist in diesem Zusammenhang 1-Butanol, welches einen wertvollen $C_4$-Baustein darstellt, dessen Bedeutung in Zukunft durch die zunehmende Verbreitung biosynthetischer Verfahren, so genannter "grüner Prozesse", noch zunehmen wird. Darüber hinaus kann 1-Butanol auch zur Treibstoffherstellung bzw. als Treibstoff eingesetzt werden. 1-Butanol kann handelsüblichen Ottokraftstoffen in beträchtlichen Mengen zugesetzt werden, wobei die Verwendung von 1-Butanol im Vergleich mit dem Einsatz von Ethanol den Vorteil aufweist, dass Butanol einen höheren Brennwert besitzt, aber im Wesentlichen nicht hygroskopisch ist. Darüber hinaus kann Ottokraftstoff mit einem beliebigen Anteil an 1-Butanol sowie auch reines 1-Butanol in den derzeit serienmäßig gefertigten Ottomotoren verbrannt werden. Aus diesen Gründen wird die Herstellung von 1-Butanol auf Basis regenerativer, zumeist biosynthetischer Verfahren derzeit intensiv erforscht und 1-Butanol aus regenerativen Prozessen als Biotreibstoff der dritten Generation bezeichnet.

Bislang sind jedoch noch keine Verfahren verfügbar, mit welchen eine selektive Herstellung von 1-Butanol ausgehend von Ethanol in großtechnischem Maßstab wirtschaftlich sinnvoll durchführbar ist.

Die entsprechenden Aldehyde finden als solche oder aber gegebenenfalls nach weiterer Umsetzung, insbesondere Hydrierung, beispielsweise als oder in Lösemitteln, als Treibstoffe und Treibstoffzusätze, als oder in Weichmachern (Plastifizierungsmitteln) oder in Lacken und Farben Anwendung.

Höhermolekulare Alkohole werden heute hauptsächlich durch den so genannten Oxo-Prozess hergestellt. Bei diesem Prozess wird fossil hergestelltes Propen mit Synthesegas und Wasser zu höheren Alkoholen umgewandelt. Für die Reaktion wird hoher Druck benötigt und zusätzlich $CO_2$ produziert. Steigende Kosten für fossile Rohstoffe, der hohe Energieaufwand und die dadurch verursachten Treibhausgasemissionen machen eine alternative Herstellungsmethode auf Basis nachwachsender Rohstoffe wünschenswert.

Speziell die Herstellung höherer Alkohole und/oder Aldehyde aus $C_1$- und $C_2$-Bausteinen, wie beispielsweise Methanol und Ethanol, wäre besonders vorteilhaft, da diese Verbindungen einerseits in "grünen Prozessen" oftmals als Neben- oder Abfallprodukte anfallen, aber andererseits auch selektiv und gezielt aus regenerativen Rohstoffen hergestellt werden können.

So kann beispielsweise Lignocellulose durch thermische und/oder chemische und anschließende enzymatische Behandlung in Zucker gespalten werden, welche von Mikroorganismen zu Ethanol vergoren werden können. Die Verwendung von Lignocellulose besitzt darüber hinaus den Vorteil, dass die holzigen Bestandteile von Pflanzen der weiteren Verwertung zugänglich gemacht werden können, welche sich nicht zur Herstellung von Nahrungsmitteln eignen. Die Verwertung von Lignocellulose führt folglich nicht zu einer konkurrierenden Verwendung wertvoller Nahrungs- und Futtermittelpflanzen zur Energiegewinnung bzw. zur chemischen Synthese; vielmehr können die beim Nahrungsmittelanbau entstehenden Reststoffe, insbesondere nicht verwertbare Pflanzenbestandteile, der weiteren Wertschöpfung zugeführt werden.

Daher hat es im Stand der Technik nicht an Versuchen gefehlt, diese Verbindungen mit unterschiedlichen Verfahren zu synthetisieren:

Eine Möglichkeit, den höheren Alkohol Butanol auf Basis nachwachsender Rohstoffe herzustellen, ist die so genannte ABE-Synthese. Dabei wird aus Biomasse fermentativ eine Mischung aus Aceton, Butanol und Ethanol hergestellt. Ein typisches Verhältnis der Stoffmengen zueinander ist 3/6/1. Bei diesem Prozess fällt Butanol jedoch häufig zusammen mit Nebenprodukten der Synthese stark verdünnt in Wasser an. Diese Mischung muss letztlich prozessintensiv aufgereinigt und Wasser energetisch aufwändig abgetrennt werden. Weiter entstehen während der Vergärung auch große Mengen an $CO_2$ sowie das noch weitaus klimaschädlichere Methan.

Neben diesen beiden bekannten Verfahren sind Ansätze mittels heterogener Katalyse bekannt, um aus den Alkoholen Ethanol oder Methanol höhere Alkohole zu produzieren. So offenbart die EP 1 829 851 A1 einen Katalysator auf Basis von Hydroxyapatit, mit welchem drucklos bei Temperaturen bis 400 °C vor allem Butanol hergestellt werden kann. Nachteilig sind hierbei vor allem die geringe Selektivität in Bezug auf die Umsetzung zu Alkoholen bei höheren Temperaturen und das Auftreten aromatischer Verbindungen und Butadien sowie ein geringer Umsatz bei niedrigeren Temperaturen und eine geringe Raum-Zeit-Ausbeute. Weiter nachteilig ist die erforderliche starke Verdünnung der Edukte bzw. Reaktanden mit Inertgas.

Darüber hinaus ist eine Reihe weiterer Verfahren bekannt, welche insbesondere in den internationalen Patentanmeldungen WO 2009/026518 A1, WO 2009/026483 A1, WO 2009/026501 A1, WO 2009/026506 A1, WO 2009/026523 A1, WO 2009/097310 A1, WO 2009/026510 A1 und WO 2009/097312 A1 offenbart sind und jeweils Hydrotalcit als Katalysator nutzen, um aus Ethanol sowie Ethanol/Methanol-Mischungen höhere Alkohole herzustellen. Nachteilig hierbei sind der relativ geringe Umsatz sowie die geringe Raum-Zeit-Ausbeute und die erforderliche starke Verdünnung der Reaktanden mit Inertgas, welche gegen ein wirtschaftlich arbeitendes Verfahren sprechen.

Die wissenschaftliche Publikation von Olson et al. "Higher-Alcohols Biorefinery - Improvement of Catalyst for Ethanol Conversion", 2004, Appl. Biochem. Biotechnol. Vol. 113-116, Seiten 913-932, beschreibt einen auf Aktivkohle basierten Katalysator, wobei mit alkalischen Promotoren getränkte Aktivkohlen mit BET-Oberflächen im Bereich von 20 bis 100 $m^2/g$ als Katalysatoren eingesetzt werden. Diese Aktivkohlekatalysatoren sind jedoch bei der Synthese von Alkoholen bzw. Aldehyden aus Ethanol bzw. Methanol nicht stabil und deaktivieren schnell. Aufgrund der kurzen Lebensdauer der Katalysatoren ist ihr Einsatz in großtechnischen Prozessen nicht möglich.

Die zuvor geschilderten Verfahren des Standes der Technik besitzen allesamt den Nachteil, dass sie höhere Alkohole bzw. Aldehyde in lediglich geringen Ausbeuten, insbesondere in geringen Raum-Zeit-Ausbeuten, liefern, so dass diese Verfahren wenig effizient und nicht ökonomisch sinnvoll durchführbar sind. Zudem ist bei den Verfahren des Standes der Technik eine aufwendig zu realisierende Prozessführung unter Verdünnung der Edukte bzw. Reaktanden mit Inertgas erforderlich. Die meisten der zuvor beschriebenen Verfahren des Standes der Technik verwenden Katalysatorsysteme mit unter großtechnischen Bedingungen unzureichenden Standzeiten der Katalysatoren. Auch ist es oftmals schwierig, kontrollierbare Reaktionsbedingungen zu schaffen, so dass verlässlich reproduzierbare Ausbeuten und Produktgemische erhältlich sind. Die meisten Verfahren sind für großtechnische Anwendungen nicht geeignet.

EP 1 052 234 offenbart die Synthese von Ethylen, Acetaldehyd, Dimethylether, 1-Butanol, 1,3-Butadien oder Benzin mit hoher Oktanzahl. Der dabei verwendete Katalysator ist ein niederkristallinischer Hydroxyapatit mit einer spezifischen Oberfläche von mindestens 2 $m^2/g$ und einem Ca/P-Mol-Verhältnis von 1,55 bis 1,8, wobei außerdem ein Metall oder Metalloxid ausgewählt aus der Gruppe Ba, Na, K, Li, Cs, Sr, Y, Ce, Sb, Eu, Ti, W und Zr anwesend ist.

Die Aufgabe der vorliegenden Erfindung besteht somit darin, ein Verfahren bereitzustellen, welches sich zur Herstellung höherer Alkohole bzw. Aldehyde aus niedermolekularen Edukten bzw. Präkursoren eignet und die zuvor geschilderten Nachteile des Standes der Technik zumindest im Wesentlichen vermeidet oder aber wenigstens verringert bzw. abschwächt.

Zur Lösung der zuvor geschilderten Aufgabe schlägt die vorliegende Erfindung ein Verfahren nach Anspruch 1 vor; weitere vorteilhafte Ausgestaltungen sind Gegenstand der diesbezüglichen abhängigen Ansprüche.

Schließlich ist weiterer Gegenstand der vorliegenden Erfindung die Verwendung eines Katalysators für die katalytische Kettenverlängerung von Kohlenstoffverbindungen nach Anspruch 14.

Es versteht sich von selbst, dass im Folgenden besondere Ausgestaltungen und Ausführungsformen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Bei allen nachstehend genannten relativen bzw. prozentualen, insbesondere gewichtsbezogenen Mengenangaben, ist zudem zu beachten, dass diese im Rahmen der vorliegenden Erfindung vom Fachmann derart auszuwählen sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % resultieren. Dies versteht sich für den Fachmann aber von selbst.

Im Übrigen gilt, dass der Fachmann anwendungsbezogen oder einzelfallbedingt von den nachfolgend aufgeführten Mengenangaben abweichen kann, ohne dass er den Rahmen der vorliegenden Erfindung verlässt.

Gegenstand der vorliegenden Erfindung ist somit - gemäß einem **ersten** Aspekt der vorliegenden Erfindung - ein Verfahren zur Herstellung höherer Alkohole und/oder Aldehyde sowie deren Gemischen durch katalytische Umsetzung von Ethanol, wobei die Umsetzung in Gegenwart mindestens eines Katalysators erfolgt, wobei der Katalysator ein Aktivkohlesubstrat, welches mit mindestens einem Metall versehen ist, umfasst, wobei der

Katalysator mindestens ein einwertiges Metall M in Form eines Alkalimetalls und mindestens ein zweiwertiges Metall M in Form von Calcium und/ oder Magnesium aufweist und wobei der Katalysator Phosphor in Form von Phosphaten enthält, wobei die folgenden molaren Verhältnisse gelten:

(i) $0{,}5 \le M^I/M^{II} \le 5$; und
(ii) $2 \le M^{II}/P \le 30$; und
(iii) $1 \le M^I/P \le 60$,

wobei der Katalysator die folgenden Mengenanteile der nachfolgend genannten Komponenten umfasst, jeweils bezogen auf den Katalysator:

(i) $M^I$: 0,1 bis 20 Gew.-%; und
(ii) $M^{II}$ in Form von Calcium und/oder Magnesium: 0,1 bis 20 Gew.-%; und
(iii) P in Form von Phosphat, berechnet als-Phosphor P: 0,01 bis 5 Gew.-%.

Mit anderen Worten weist der Katalysator bzw. das Aktivkohlesubstrat erfindungsgemäß eine Metalldotierung auf. Wie nachfolgend noch eingehend geschildert, kann die Ausrüstung bzw. Dotierung des Katalysators bzw. des Aktivkohlesubstrats entweder im Verlauf der Katalysatorherstellung oder aber alternativ hierzu nachträglich erfolgen, wobei es sich im Rahmen der vorliegenden Erfindung als vorteilhaft erwiesen hat und besonders wirksame Katalysatoren erhalten werden, wenn die Ausrüstung bzw. Dotierung des Katalysators bzw. des Aktivkohlesubstrats im Verlauf der Katalysatorherstellung vorgenommen wird.

Denn, wie die Anmelderin in überraschender Weise herausgefunden hat, sind höhere Alkohole und/oder Aldehyde sowie deren Gemische in einfacher und effizienter Weise durch ein Verfahren zugänglich, bei welchem Ethanol in Gegenwart eines Katalysators auf Basis eines mit einem Metall versehenen Aktivkohlesubstrats umgesetzt wird. Das erfindungsgemäße Verfahren ermöglicht insbesondere die selektive Herstellung höherer Alkohole bzw. Aldehyde mit sehr guten Ausbeuten und ist darüber hinaus in großtechnischem Maßstab wirtschaftlich durchführbar. Darüber hinaus kann das erfindungsgemäße Verfahren unter kontrollierten Bedingungen beliebig reproduziert werden.

Das erfindungsgemäße Verfahren geht mit einer Vielzahl von Vorteilen einher, von denen zumindest wesentliche in nicht abschließender Weise nachfolgend genannt werden sollen:

Das erfindungsgemäße Verfahren besitzt hohe Selektivität hinsichtlich der Bildung von Alkoholen und Aldehyden, wobei insbesondere besonders hohe Selektivitäten hinsichtlich der Bildung von $C_3$-$C_6$-Alkohlen bzw. $C_3$-$C_6$-Aldehyden erreicht werden können. Bei Verwendung eines stark basischen Katalysators kann insbesondere die Bildung von Butadien und aromatischen Verbindungen unterdrückt werden. Die hohe Selektivität des erfindungsgemäßen Verfahrens erlaubt eine einfache und kostengünstige Reinigung, Fraktionierung und anschließende Weiterverarbeitung des erhaltenen Produktgemisches.

Mit dem erfindungsgemäßen Verfahren können deutlich höhere Raum-Zeit-Ausbeuten für Alkohole und Aldehyde als mit Verfahren des Standes der Technik erzielt werden.

Die Verwendung eines Katalysators auf Basis eines Aktivkohlesubstrats erlaubt aufgrund der hohen spezifischen Oberfläche der Aktivkohle eine höhere Alkoholbelastung im Zustrom (ausgedrückt in kg Alkohol pro kg Katalysator und Stunde) als bislang üblich. Gleichzeitig muss keine oder allenfalls nur eine geringfügige Anreicherung der Reaktanden mit Inertgas vorgenommen werden. Dies führt gleichfalls dazu, dass das erfindungsgemäße Verfahren in großtechnischem Maßstab und aus ökonomischer Sicht sehr günstig bzw. effizient durchgeführt werden kann.

Die Herstellungskosten für einen Katalysator auf Basis eines erfindungsgemäß eingesetzten metallbeladenen Aktivkohlesubstrats sind deutlich geringer als für herkömmliche Übergangsmetallkatalysatoren, insbesondere wenn die Dotierung bzw. Ausrüstung des Aktivkohlesubstrats durch eine Imprägnierung der Aktivkohle vor oder nach der Aktivierung der Aktivkohle erzielt wird. Die Dotierung ermöglicht eine molekulare und insbesondere besonders homogene Verteilung der Aktivkomponenten, wodurch mit einem geringen Materialeinsatz an Metallverbindungen sehr leistungsfähige Katalysatoren bereitgestellt werden können.

Die katalytische Oberfläche des resultierenden Katalysators besteht aus einer Kombination der Eigenschaften des kohlenstoffhaltigen Trägermaterials und der speziellen Dotierung. Die katalytischen Eigenschaften sind in weiten Bereichen durch einfache Variation von Kohlenstoffquelle und Dotierung gezielt steuerbar und/oder einstellbar, wodurch eine hohe Flexibilität in der Zusammensetzung des erhaltenen Produktgemisches erreicht wird. Insbesondere ist das Verhältnis von linearen zu verzweigten Alkoholen oder Aldehyden gezielt bzw. selektiv steuerbar.

Im Rahmen des erfindungsgemäßen Verfahrens werden erstmals mit Metallen bzw. Metallverbindungen dotierte basische Aktivkohlekatalysatoren eingesetzt, welche es erlauben, bei guter Standzeit gleichzeitig mit hoher Aktivität und hoher Raum-Zeit-Ausbeute zu arbeiten. Weiterhin vorteilhaft sind das Ausbleiben der Bildung von Butadien und die geringe Selektivität zu aromatischen Nebenprodukten im Vergleich zum Stand der Technik.

Das erfindungsgemäße Verfahren ist als eigenständiges Verfahren zur Herstellung höherer Alkohole und Aldehyde konzipiert. Es kann jedoch auch mit einer Oxo-Synthese (Oxo-Prozess) gekoppelt werden, wodurch im Rahmen der Oxo-Synthese einerseits die Rohstoffkosten und Katalysatorverbrauchskosten gesenkt werden sowie andererseits eine neue Rohstoffbasis erschlossen wird. Die Rohstoffkosten können insbesondere gesenkt werden, da- anstatt immer teurer werdenden Propylens oder längerkettiger Alkene - kostengünstigeres Methanol und/oder Ethanol eingesetzt werden können und dennoch vergleichbare Produktgemische und Komponenten erhalten werden. Die Katalysatorverbrauchskosten pro Masse an erzeugtem Alkohol bzw. Aldehyd werden im Vergleich zur Oxo-Synthese gesenkt, da anstelle von teuren Rhodium- und Cobaltkomplexverbindungen sehr viel günstigere metallbeladene, im Allgemeinen basisch eingestellte Aktivkohlekatalysatoren eingesetzt werden können.

Wie bereits eingangs erwähnt, werden im Rahmen der vorliegenden Erfindung unter den Begriffen "höherer Alkohol" und "höherer Aldehyd" organische Verbindungen mit der zuvor definierten Anzahl an Kohlenstoffatomen und mindestens einer Hydroxy- bzw. Aldehydfunktion verstanden. Hierbei kann es sich sowohl um verzweigte als auch um lineare oder aber um zyklische Kohlenstoffketten bzw. -gerüste oder aber um aromatische bzw. teilweise aromatische Systeme handeln. Bevorzugt werden im Rahmen der vorliegenden Erfindung lineare und/oder verzweigte Ketten bzw. Gerüste von Kohlenstoffatomen erhalten.

Es hat sich erfindungsgemäß als besonders vorteilhaft erwiesen, wenn die höheren Alkohole und/oder Aldehyde ausgewählt sind aus linearen und/oder verzweigten Alkohohlen und/oder Aldehyden.

Im Allgemeinen sind die höheren Alkohole und/oder Aldehyde im Rahmen der vorliegenden Erfindung ausgewählt aus der Gruppe von $C_3$-$C_{30}$-Alkoholen und/oder $C_3$-$C_{30}$-Aldehyden, insbesondere $C_3$-$C_{20}$-Alkoholen und/oder $C_3$-$C_{20}$-Aldehyden, vorzugsweise $C_3$-$C_{15}$-Alkoholen und/oder $C_3$-$C_{15}$-Aldehyden, sowie deren Gemischen.

Bevorzugt werden im Rahmen der vorliegenden Erfindung primäre Alkohole und Aldehyde mit einer Kettenlänge von drei bis sechs Kohlenstoffatomen erhalten, wobei den $C_3$- und $C_4$-Verbindungen, insbesondere 1-Butanol bzw. n-Butanol, besondere technische Bedeutung zukommt. Im Rahmen der vorliegenden Erfindung ist es möglich, diese kurzkettigen Produkte mit besonders hohen Selektivitäten herzustellen, wobei es gleichfalls möglich ist, die Bildung von aromatischen Produkten weitestgehend zu unterdrücken.

Das erfindungsgemäß erhaltene Produktgemisch kann entweder in einzelne Fraktionen oder in einzelne Verbindungen aufgetrennt werden, welche dann beispielsweise als Lösemittel, Treibstoffe oder Treibstoffzusätze oder Edukte bzw. Präkursoren zur Herstellung beispielsweise von Weichmachern und Additiven für Lacke und Farben oder auch Kunststoffen verwendet werden können.

Darüber hinaus hat es sich im Rahmen der vorliegenden Erfindung als besonders vorteilhaft erwiesen, wenn bei der Durchführung des erfindungsgemäßen Verfahrens ein Butanol(e) enthaltendes Produktgemisch resultiert. Das erfindungsgemäße Verfahren kann nämlich insbesondere mit einer besonders hohen Selektivität in Bezug auf die Bildung von Butanol, insbesondere n-Butanol bzw. 1-Butanol, durchgeführt werden.

Im Rahmen der vorliegenden Erfindung kann Ethanol als Reinstoff, gegebenenfalls in Gegenwart eines Inertgases, insbesondere Stickstoff, umgesetzt werden. Es ist jedoch auch möglich, dass Ethanol als Gemisch von Ethanol mit mindestens einem weiteren Alkohol, vorzugsweise Methanol, und/oder mit mindestens einem Aldehyd, gegebenenfalls in Gegenwart eines Inertgases, insbesondere Stickstoff, umgesetzt wird. Im Rahmen der vorliegenden Erfindung ist es jedoch auch möglich, dass Ethanol bzw. die gegebenenfalls weiteren vorhandenen Alkohole und Aldehyde nicht als Reinstoffe eingesetzt werden müssen. Das heißt, dass im Rahmen der vorliegenden Erfindung Alkohole und Aldehyde in jeweils technischer Qualität - also mit einem gewissen Anteil an Verunreinigungen - eingesetzt werden können. Insbesondere müssen die eingesetzten Alkohole und Aldehyde nicht absolutiert sein; vielmehr können sie einen gewissen, nicht unerheblichen Anteil an Wasser aufweisen. So kann beispielsweise ein Ethanol/Wasser-Azeotrop, welches 96 Vol.-% Ethanol enthält, ohne weitere Vorbehandlung bzw. Reinigung zur Durchführung des erfindungsgemäßen Verfahrens eingesetzt werden.

Vorteilhafterweise wird Ethanol in volumenbezogenen Mengen von 5 bis 100 Vol.-%, insbesondere 10 bis 100 Vol.-%, vorzugsweise 20 bis 100 Vol.-%, besonders bevorzugt 25 bis 100 Vol.-%, bezogen auf die Ausgangsmischung, eingesetzt. Sofern Ethanol nicht zu 100 Vol.-%, (d. h. mit anderen Worten nicht als Reinstoff) eingesetzt wird, kann der verbleibende Volumenanteil der Mischung der Edukte durch Inertgas(e), vorzugsweise Stickstoff, und/oder mindestens einen weiteren Alkohol, vorzugsweise Methanol, und/oder mindestens einen Aldehyd gebildet sein.

Insbesondere ist es möglich, das erfindungsgemäße Verfahren mit einem Ethanol/Inertgas-Gemisch, welches mindestens 5 Vol.-% Ethanol enthält, durchzuführen. Gleichfalls kann Ethanol auch als Reinstoff, d. h. mit 100 Vol.-%, eingesetzt werden. Hierin ist einer der Vorteile des erfindungsgemäßen Verfahrens zu sehen, da es den Einsatz unverdünnter Eduktströme ermöglicht, was mit den Verfahren des Standes der Technik bislang nicht erreicht werden kann.

Gemäß dem Stand der Technik muss dem umzusetzenden Ethanol stets ein Inertgas - üblicherweise Stickstoff - beigemischt werden, wobei oftmals der volumenbezogene Anteil des Inertgases größer ist als der volumenbezogene Anteil des Ethanols. Gemäß den Verfahren des Standes der Technik wird häufig ein Trägergas eingesetzt, wodurch sich die Produktionskosten drastisch erhöhen. Einerseits fallen Kosten für das Inertgas an, und andererseits muss das Inertgas mit dem bzw. den Edukten aufgeheizt und mit den Produkten wieder abgekühlt werden, wodurch zusätzliche Kosten durch den Energieverbrauch entstehen, welche bis zu etwa 20 % oder sogar mehr der gesamten Verfahrenskosten ausmachen können. Darüber hinaus setzt die Verdünnung des Edukts bzw. des Eduktgemisches mit einem Inert- bzw. Trägergas die Raum-Zeit-Ausbeute des gesamten Verfahrens deutlich herab und mindert folglich die Effizienz des Verfahrens, wobei gleichzeitig die Verfahrenskosten nochmals gesteigert werden.

Darüber hinaus bietet das erfindungsgemäße Verfahren den Vorteil, dass es - im Vergleich zum Stand der Technik - mit Edukten lediglich technischer Qualität durchgeführt werden kann, welche kostengünstig zu beziehen sind, da sie nicht speziell behandelt bzw. gereinigt sind.

Ferner kann erfindungsgemäß durch die gezielte Wahl der Edukte auch die Selektivität in der Bildung der Produkte beeinflusst werden. So führt die alleinige Verwendung von Ethanol als Edukt, gegebenenfalls in Kombination mit einem Inertgas, zu überwiegend linearen Produkten, d. h. Produkten mit unverzweigten Kohlenstoffketten; gleichzeitig wird eine hohe Selektivität des gesamten Verfahrens hin zur Bildung von Butanol bzw. von $C_4$-Produkten beobachtet. Produkte mit verzweigten Kohlenstoffketten treten dagegen erst bei einer Kettenlänge von mehr als vier Kohlenstoffatomen auf, wobei der Anteil dieser Produkte am Gesamtproduktgemisch äußerst gering ist.

Werden hingegen beispielsweise Ethanol/Methanol-Gemische eingesetzt, so wird auch eine bevorzugte Bildung von $C_3$-und $C_4$-Produkten, insbesondere Propanol und Butanole (d. h. iso-Butanol und n-Butanol), beobachtet, wobei die Selektivitäten in Bezug auf die einzelnen Produkte geringer sind und der Anteil verzweigter Produkte erhöht ist.

Doch nicht nur die Wahl der Edukte, sondern auch die weiteren Prozessparameter besitzen einen großen Einfluss auf die Effizienz und Selektivität des erfindungsgemäßen Verfahrens sowie auf die Ausbeute und die Produktverteilung:

Im Allgemeinen erfolgt die Umsetzung im Rahmen des erfindungsgemäßen Verfahrens in der Gasphase. Dabei hat es sich als besonders vorteilhaft erwiesen, wenn die Umsetzung oberhalb der Siedetemperaturen der Edukte und/oder der Produkte, vorzugsweise oberhalb der Siedetemperaturen der Edukte und Produkte, erfolgt.

Besonders gute Umsätze, Ausbeuten und Selektivitäten werden erhalten, wenn die Umsetzung bei Temperaturen im Bereich von 150 °C bis 600 °C, insbesondere 250 bis 450 °C, vorzugsweise 300 bis 400 °C, durchgeführt wird.

Weiterhin kann im Rahmen der vorliegenden Erfindung die Umsetzung bei reduziertem Druck, Normaldruck oder bei Überdruck durchgeführt werden. In diesem Zusammenhang hat es sich als vorteilhaft erwiesen, wenn die Umsetzung bei einem Absolutdruck im Bereich von Atmosphärendruck bis 100 bar, insbesondere im Bereich von Atmosphärendruck bis 50 bar, vorzugsweise im Bereich von Atmosphärendruck bis 25 bar, durchgeführt wird.

Auch die Reaktionsdauern bzw. Kontaktzeiten besitzen einen großen Einfluss auf Umsätze und Ausbeuten einerseits und die Selektivität der Produktbildung andererseits. Im Allgemeinen wird die Umsetzung mit Reaktionsdauern und/oder Kontaktzeiten im Bereich von 0,001 bis 120 Sekunden, insbesondere 0,01 bis 60 Sekunden, vorzugsweise 0,05 bis 30 Sekunden, durchgeführt.

Unter dem Begriff "Kontaktzeit" ist im Rahmen der vorliegenden Erfindung insbesondere der Quotient aus dem Volumen des eingesetzten Katalysators und dem Volumenstrom des eingesetzten Eduktgases bzw. Eduktgasgemisches zu verstehen.

Durch Variation der oben genannten Parameter können die mit dem erfindungsgemäßen Verfahren zu erzielenden Umsätze, Ausbeuten und Selektivitäten gezielt an die jeweiligen Anforderungen angepasst bzw. optimiert und/oder gezielt gesteuert werden. So kann insbesondere durch geeignete Abstimmung von Temperatur und Druck jeweils das Optimum hinsichtlich der Umsätze, Ausbeuten und Selektivitäten gezielt eingestellt werden, wobei sich eine Temperaturerhöhung im Allgemeinen positiv auf die Umsätze und Ausbeuten auswirkt, die Überschreitung eines gewissen Temperaturbereichs die Ausbeuten und insbesondere die Selektivität der Produktbildung jedoch vermindert. Eine Erhöhung des Drucks ermöglicht hingegen im Allgemeinen gesteigerte Umsätze, Ausbeuten und Selektivitäten bei niedrigeren Temperaturen.

Durch geeignete Abstimmung der Kontaktzeiten, welche die Verweilzeit der Substanzen im Reaktor und somit die Reaktionszeit bestimmen, können die Umsätze, Ausbeuten und Selektivitäten noch weiter optimiert werden. Hierbei müssen die Kontaktzeiten einerseits ausreichend lang sein, um gute Umsätze und Ausbeuten zu gewährleisten, dürfen andererseits jedoch nicht übermäßig lang gewählt sein, um die Bildung von Nebenprodukten möglichst zu vermeiden. Bei zu langen Kontaktzeiten nimmt nämlich die Bildung von Nebenprodukten stark zu, wodurch die Selektivität des Verfahrens gesenkt wird.

Im Allgemeinen tritt bei kurzen Kontaktzeiten die Bildung von Alkoholen und bei längeren Kontaktzeiten auch die Bildung von Aldehyden auf.

Das Verfahren nach der Erfindung kann grundsätzlich diskontinuierlich, d. h. chargenweise bzw. im Batchbetrieb, oder aber kontinuierlich betrieben werden. Bevorzugt, insbesondere bei technischer oder industrieller Anwendung, ist eine kontinuierliche Verfahrensführung, welche hohe Raum-Zeit-Ausbeuten und Umsätze ermöglicht und folglich besonders wirtschaftlich durchgeführt werden kann.

Im Allgemeinen wird das erfindungsgemäße Verfahren mit einer Raum-Zeit-Ausbeute, angegeben als Menge aller gebildeter Produkte pro Katalysatorvolumen und pro Zeiteinheit, im Bereich von 10 bis 3.000 g/(Liter • h), insbesondere 25 bis 2.500 g/(Liter • h), vorzugsweise 30 bis 2.000 g/(Liter • h), besonders bevorzugt 50 bis 1.500 g/(Liter • h), durchgeführt.

Hierbei kann das Verfahren mit einem stoffmengenbezogenen Umsatz, bezogen auf die eingesetzten Edukte, insbesondere Ethanol, im Bereich von 20 bis 90 %, insbesondere 30 bis 80 %, vorzugsweise 40 bis 75 %, durchgeführt werden.

Der stoffmengenbezogene Umsatz ist dabei insbesondere definiert als Quotient aus der Stoffmenge an umgesetztem Edukt und eingesetztem Edukt, d. h.

$$\text{Umsatz} = (\text{umgesetztes Edukt [mol]}) / (\text{eingesetztes Edukt [mol]}) \cdot 100 = (\text{eingesetztes Edukt [mol]} - \text{nicht umgesetztes Edukt [mol]}) / (\text{eingesetztes Edukt [mol]}) \cdot 100.$$

Wie oben bereits dargelegt, bewirkt eine Steigerung des Drucks und/oder der Temperatur im Allgemeinen eine Erhöhung der Umsätze, wobei aber die Erhöhung des Umsatzes ab einem gewissen Punkt auf Kosten der Selektivität erreicht wird, so dass für jeden Einzelfall die optimale Abstimmung der einzelnen Prozessparameter aufeinander bestimmt werden muss.

Die oben angegebenen Raum-Zeit-Ausbeuten und stoffmengenbezogenen Umsätze beschreiben Bereiche, in welchen das erfindungsgemäße Verfahren besonders wirtschaftlich und nach verfahrensökonomischen Gesichtspunkten günstig durchgeführt werden kann, wobei eine hohe Selektivität in den erhaltenen Produktgemischen erzielt wird.

In diesem Zusammenhang kann es weiterhin vorgesehen sein, dass das Verfahren mit einer stoffmengenbezogenen Selektivität, bezogen auf $C_4$-Produkt(e), insbesondere Butanol, und berechnet als prozentuales Verhältnis von Stoffmenge an $C_4$-Produkt(en) zu der umgesetzten Stoffmenge an Edukt(en), im Bereich von 5 bis 70 %, insbesondere 10 bis 60 %, vorzugsweise 10 bis 50 %, durchgeführt wird.

Diese hohen Selektivitäten sind mit dem erfindungsgemäßen Verfahren problemlos erreichbar, wodurch sich das erfindungsgemäße Verfahren weiterhin gegenüber Verfahren des Standes der Technik auszeichnet. Die stoffmengenbezogene Selektivität ist hierbei insbesondere definiert als Quotient aus der Kohlenstoffmenge des jeweiligen Produkts, insbesondere Kohlenstoffmenge an $C_4$-Produkten, und der Kohlenstoffmenge an umgesetztem Edukt (Selektivität Komponente i = (Kohlenstoffmenge Komponente i im Produktgemisch [mol]) / (Kohlenstoffmenge umgesetztes Edukt [mol]) • 100, insbesondere Selektivität $C_4$-Produkte = (Kohlenstoffmenge $C_4$-Produkt [mol]) / (Kohlenstoffmenge umgesetztes Edukt [mol]) • 100).

Weiterhin kann das erfindungsgemäße Verfahren, insbesondere mit einer stoffmengenbezogenen Ausbeute bezogen auf $C_4$-Produkt(e), insbesondere Butanol, und berechnet als das Produkt aus stoffmengenbezogenem Umsatz und stoffmengenbezogener Selektivität (wie oben definiert) im Bereich von 5 bis 50 %, insbesondere 5 bis 40 %, vorzugsweise 5 bis 30 %, durchgeführt werden.

Die stoffmengenbezogene Ausbeute der Komponente i ist dabei insbesondere definiert als das Produkt aus dem stoffmengenbezogenen Umsatz der Komponente i und der stoffmengenbezogenen Selektivität der Komponente i (Ausbeute Komponente i = Umsatz (i) • Selektivität (i), insbesondere Ausbeute $C_4$-Produkte = Umsatz $C_4$-Produkte • Selektivität $C_4$-Produkte).

Die zuvor bezeichneten stoffmengenbezogenen Selektivitäten und Ausbeuten sind charakteristisch für das erfindungsgemäße Verfahren und zeichnen dieses gegenüber den Verfahren des Standes der Technik aus.

Wie oben bereits ausgeführt, kommt im Rahmen des erfindungsgemäßen Verfahrens ein Katalysator auf Basis eines metallbeladenen Aktivkohlesubstrats zum Einsatz.

Die im Rahmen der vorliegenden Erfindung eingesetzte Aktivkohle enthält vorzugsweise nicht nur Kohlenstoff, sondern auch kleine Mengen an Sauerstoff, Stickstoff, Schwefel und Wasserstoff, welche chemisch in Form verschiedener funktioneller Gruppen, wie etwa Carbonyl-, Carboxyl-, Phenol-und Ethergruppen sowie Lactonen und Chinonen gebunden sind. Diese Oberflächenoxide können aus den Rohstoffen resultieren, oder aber sie können durch den Aktivierungsprozess, durch den Einfluss von chemischen Aktivatoren sowie durch den Einfluss von Sauerstoff oder Wasserdampf entstehen. Die chemischen Eigenschaften der Oberfläche spielen eine signifikante Rolle für die Adsorption und die Katalyse.

Die Ausgangsmaterialien für Aktivkohle, welche zur Herstellung von erfindungsgemäß einsetzbaren Katalysatoren geeignet sind, besitzen im Allgemeinen mineralische Komponenten, die während des Aktivierungsprozesses aufkonzentriert werden können. Weiterhin ist es auch möglich, dass anorganische Chemikalien für die Aktivierung der Aktivkohle nicht vollständig entfernt werden oder ganz auf der Aktivkohle verbleiben.

Der Aschegehalt von Aktivkohlen wird maßgeblich durch die mineralischen Komponenten bestimmt. Die Hauptbestandteile dieser Asche sind Alkali- und Erdalkalimetalle, zumeist in Form von Carbonaten und Phosphaten, gegebenenfalls zusammen mit Kieselsäure sowie Eisen- und Aluminiumoxiden. Der Aschegehalt von Aktivkohlen kann durch Waschen mit Wasser oder Säure reduziert werden. Kommerzielle Produkte weisen daher Aschegehalte von unter einem bis zu zwanzig Prozent auf.

Aktivkohle wirkt zugleich als Katalysator und als Katalysatorträger: Die katalytische Aktivität der Aktivkohle als solcher beruht im Wesentlichen auf der Struktur des Kohlenskeletts, die aus einer Mischung von amorphem und graphitähnlichem Kohlenstoff besteht; am Rand von Schichten gibt es viele chemisch ungesättigte Ecken und Kanten, die als so genannte Gitterlücken fungieren, und auf der internen Aktivkohleoberfläche der im Rahmen des erfindungsgemäßen Verfahrens eingesetzten Aktivkohle liegen bevorzugt die bereits genannten Oberflächenoxide, welche an Redoxreaktionen teilnehmen können und mitunter den Grund für die chemische Aktivität von Aktivkohlen darstellen. Zudem fungieren die erfindungsgemäß eingesetzten Aktivkohlen als Träger für die Metalldotierung.

Der zur Durchführung des erfindungsgemäßen Verfahrens eingesetzte Katalysator und/oder das eingesetzte Aktivkohlesubstrat sind im Allgemeinen basisch ausgerüstet und/oder ausgebildet.

Gemäß der vorliegenden Erfindung wird die basische Ausrüstung durch Carbonate und/oder Phosphate, besonders bevorzugt durch Carbonate und Phosphate, wie in Anspruch 1 beansprucht, bereitgestellt.

Die basische Ausrüstung kann dabei bei der Herstellung des Katalysators oder aber nachträglich, insbesondere mittels Imprägnierung, erfolgen. Besonders gute Ergebnisse werden im Rahmen der vorliegenden Erfindung jedoch erhalten, wenn die basische Ausrüstung bei der Herstellung des Katalysators erfolgt.

Unter einer basischen Ausrüstung ist im Rahmen der vorliegenden Erfindung zu verstehen, dass der Katalysator bzw. das Aktivkohlesubstrat basische Gruppen und/oder Verbindungen oder aber basisch reagierende Gruppen und Verbindungen aufweist. Entscheidend ist, dass der basische Charakter dieser Gruppen bzw. Verbindungen im fertig hergestellten Katalysator unter Reaktionsbedingungen erhalten bleiben. Dabei ist es durchaus möglich, dass die ursprünglich eingesetzten Verbindungen bei der Katalysatorherstellung oder aber bei der Katalysereaktion umgewandelt werden; in diesem Fall müssen die Umwandlungsprodukte basischen Charakter besitzen. So können beispielsweise Carbonate bei einer Aktivierung des Aktivkohlesubstrats zu Oxiden reagieren, gleichfalls ist es jedoch auch möglich, dass die Carbonate mit dem Kohlenstoffgerüst des Aktivkohlesubstrats, beispielsweise unter Bildung von Phenolaten, Oxiden, Anhydriden, Hydroxiden etc., reagieren.

Darüber hinaus sollte der erfindungsgemäß eingesetzte Katalysator eine große spezifische Oberfläche aufweisen. Der im Rahmen des erfindungsgemäßen Verfahrens eingesetzte Katalysator und/oder das Aktivkohlesubstrat weist im Allgemeinen eine spezifische Oberfläche (BET) im Bereich von 450 bis 3.000 m$^2$/g, insbesondere 500 bis 2.500 m$^2$/g, vorzugsweise 600 bis 2.250 m$^2$/g, besonders bevorzugt 900 bis 1.700 m$^2$/g, ganz besonders bevorzugt 950 bis 1.500 m$^2$/g, noch mehr bevorzugt 1.000 bis 1.350 m$^2$/g, auf.

Darüber hinaus sollte der erfindungsgemäß eingesetzte Katalysator ein großes Mikroporenvolumen besitzen. Insbesondere kann es vorgesehen sein, dass der Katalysator und/oder das Aktivkohlesubstrat ein Mikroporenvolumen, insbesondere ein Mikroporenvolumen nach Gurvich, im Bereich von 0,1 bis 3,0 ml/g, insbesondere 0,2 bis 2,5 ml/g, vorzugsweise 0,25 bis 1 ml/g, besonders bevorzugt 0,3 bis 0,7 ml/g, aufweist.

Weiterhin hat es sich im Rahmen der vorliegenden Erfindung als vorteilhaft erwiesen, wenn der Katalysator und/oder das Aktivkohlesubstrat eine Standzeit von mindestens 10 Tagen, insbesondere mindestens 20 Tagen, vorzugsweise mindestens 30 Tagen, besonders bevorzugt mindestens 6 Monaten, aufweist. Lange Standzeiten des erfindungsgemäß eingesetzten Katalysators ermöglichen die kontinuierliche großtechnische Durchführung des erfindungsgemäßen Verfahrens und ermöglichen somit eine in ökonomischer Hinsicht günstige Herstellung höherer Alkohole und Aldehyde.

Gleichfalls hat es sich als vorteilhaft erwiesen, wenn der Katalysator und/oder das Aktivkohlesubstrat mindestens eine funktionelle Gruppe, vorzugsweise eine polare und/oder ionische funktionelle Gruppe, umfasst. Dabei kann es vorgesehen sein, dass die mindestens eine funktionelle Gruppe ausgewählt ist aus Carbonyl-, Carboxylat-, Hydroxyl-, Oxid-, Ether-, Ester-, Lacton-, Phenol- und/oder Chinongruppen. Die vorgenannten funktionellen Gruppen können beispielsweise durch Reaktionen des Kohlestoffgerüsts des Aktivkohlesubstrats mit einer für die basische Ausrüstung benötigten Verbindung während der Aktivierung des Aktivkohlesubstrats gebildet werden (wie zuvor beschrieben).

Besonders gute Ergebnisse werden im Rahmen des erfindungsgemäßen Verfahrens erhalten, wenn die vorgenannten Verbindungen und/oder Substanzen in speziellen molaren Verhältnissen zueinander in dem erfindungsgemäß eingesetzten Katalysator vorhanden sind. In diesem Zusammenhang ist es im Rahmen der vorliegenden Erfindung bevorzugt, wenn für den Katalysator folgenden molaren Verhältnisse gilt:

(i) $2 \leq M^{I}/M^{II} \leq 3$; und/oder

(ii) $2 \leq M^{II}/P \leq 8$; und/oder

(iii) $5 \leq M^{I}/P \leq 10$; und/oder

(iv) $1 \leq K/Na \leq 20$, insbesondere $10 \leq K/Na \leq 20$; und/oder

(v) $1 \leq Ca/Mg \leq 10$, insbesondere $4 \leq Ca/Mg \leq 6$.

Gleichfalls werden im Rahmen des erfindungsgemäßen Verfahrens sehr gute Ergebnisse erhalten, wenn der Katalysator die folgenden Mengenanteile (Gewichtsprozente) der nachfolgend genannten Komponenten umfasst, wobei die nachfolgenden Angaben jeweils auf den Katalysator bezogen sind:

(i) $M^{I}$ insbesondere 0,2 bis 15 Gew.-%, vorzugsweise 0,5 bis 10 Gew.-%; und/oder

(ii) $M^{II}$ insbesondere 0,2 bis 10 Gew.-%, vorzugsweise 0,5 bis 5 Gew.-%; und/oder

(iii) P insbesondere 0,02 bis 2,5 Gew.-%, vorzugsweise 0,02 bis 1 Gew.-%.

Besonders gute Umsätze, Ausbeuten und Selektivitäten können im Rahmen des erfindungsgemäßen Verfahrens erzielt werden, wenn der erfindungsgemäß eingesetzte Katalysator die zuvor genannten Metalle und Phosphor sowohl in den speziellen molaren Verhältnissen zueinander als auch in den jeweiligen absoluten Stoffmengenanteilen enthält.

Insbesondere kann es im Rahmen der vorliegenden Erfindung vorgesehen sein, dass als Katalysator eine basisch ausgerüstete und/oder eingestellte Aktivkohle, welche mit mindestens einer Kalium-und Calcium- und/oder Magnesiumdotierung, versehen ist, eingesetzt wird. Hierbei hat es sich als besonders vorteilhaft erwiesen, wenn eine mit Phosphat und/oder Carbonat basisch eingestellte, mit Kalium und Calcium und/oder Magnesium dotierte Aktivkohle eingesetzt wird.

Gemäß einer besonderen Ausführungsform der vorliegenden Erfindung kann als Katalysator eine Formaktivkohle eingesetzt werden, wie sie in der DE 10 2004 033 561 A1 und der DE 10 2004 033 561 B4, deren gesamter jeweiliger Offenbarungsgehalt hiermit durch Bezugnahme vollumfänglich eingeschlossen ist.

Gemäß dieser Ausführungsform kommt als Katalysator eine Formaktivkohle zum Einsatz, welche herstellbar ist durch ein Verfahren zur Herstellung von Formaktivkohle aus einem Kohlenstoffträger, einem Bindemittel und einer katalytischen Komponente der allgemeinen Formel (I)

$$[M]_{m3}[AO_{n4}]_{m4} \qquad (I)$$

wobei

- M ein Kation bezeichnet und ausgewählt ist aus der Gruppe von Alkali-oder Erdalkalimetallkationen;

- m3 und m4 stöchiometrische Koeffizienten mit ganzen Zahlen mit $m3 \geq 1$ und $m4 \geq 1$ bezeichnen;

- $[AO_{n4}]$ ein sauerstoffhaltiges Anion mit dem ganzzahligen stöchiometrischen Koeffizienten $n4 \geq 1$ bezeichnet;

- $[AO_{n4}]$ vorzugsweise ausgewählt wird aus der Gruppe von Carbonaten oder Hydroxiden,

wobei das Bindemittel erhalten wird aus der Umsetzung eines wasserlöslichen kohlenhydrathaltigen Ausgangsstoffes mit einem Glucosegehalt von $\geq 50$ Gew.-%, insbesondere von $\geq 60$ Gew.-%, wobei der Kohlenstoffträger zunächst mit der katalytischen Komponente vermischt wird, wobei anschließend die Mischung aus katalytischer Komponente und Kohlenstoffträger mit dem Bindemittel vermischt wird, wobei die so erhaltene Mischung aus Kohlenstoffträger, katalytischer Komponente und Bindemittel zu Formlingen verpresst wird und wobei die Formlinge carbonisiert und aktiviert werden, wobei das Bindemittel aus der Umsetzung des kohlenhydrathaltigen Ausgangsstoffes mit einem Zuschlagstoff erhalten wird, wobei zum Erhalt des Bindemittels der Zuschlagstoff dem kohlenhydrathaltigen Ausgangsstoff zugegeben wird vor der Vermischung des Bindemittels mit der Mischung aus dem Kohlenstoffträger und der katalytischen Komponente; dabei kann der Zuschlagstoff insbesondere ausgewählt sein kann aus der Gruppe von Phosphorsäuren und/oder deren Salzen, Schwefelsäuren und/oder deren Salzen und/oder Schwefelsäurederivaten und/oder deren Salzen.

Dieses allgemeine Herstellungsverfahren und die chemische Zusammensetzung der Verbindungen der Metalle, Übergansmetalle und Seltenen Erden in der Dotierung zielen ursprünglich auf die Adsorption von sauren Gasen ab. Zur Herstellung eines im erfindungsgemäßen Verfahren einsetzbaren, besonders wirkungsvollen Katalysators kann dieses Herstellungsverfahren noch geringfügig adaptiert werden, wobei die Adaptionen vollumfänglich in der DE 10 2004 033 561 A1 bzw. der DE 10 2004 033 561 B4 offenbart sind.

Als Dotierungsreagenz für die Alkoholsynthese werden für die Formaktivkohle für die Alkohol- bzw. Aldehydsynthese Metallsalze eingesetzt, deren Kationen gemäß Anspruch 1 ausgewählt sind aus den Metallen der 1. und 2. Hauptgruppe. Vorzugsweise wird dem Kohlenstoffträger $K_2CO_3$ als Aktivator zugesetzt. Kaliumcarbonat reagiert mit dem Kohlenstoffträger unter anderem unter Kohlenstoffverbrauch und führt zur Bildung von sehr kleinen Mikroporen, die während der Gasaktivierung mit Wasserdampf weiter zu größeren Mikroporen und Mesoporen aufgeweitet werden und so zum gewünschten Porensystem führen. Durch Variation der Menge an $K_2CO_3$ in dem Kohlenstoffträger und der Aktivierungsbedingungen (Temperatur, Wasserdampfmenge, Verweilzeit etc.) lassen sich deswegen unterschiedliche Porengrößen und Porenverteilungen in der Formaktivkohle einstellen.

Die vorgesehenen Zuschlagstoffe, wie etwa $K_2CO_3$, müssen noch vor der Vermischung des Bindemittels mit dem Kohlenstoffträger dem kohlenhydrathaltigen Ausgangsstoff des Bindemittels zugegeben werden. Das Bindemittel zur Herstellung von Formaktivkohle ist erhältlich aus der Umsetzung eines wasserbasierten glucosehaltigen Ausgangsstoffes mit einem Zuschlagstoff, wobei der Zuschlagstoff ausgewählt ist aus der Gruppe der Phosphorsäuren und/oder deren Salzen. Bei dem wasserbasierten glucosehaltigen Ausgangsstoff handelt es sich vorzugsweise um Glucose oder Glucosederivate, wie vorzugsweise Glucosesirup, Dicksaft oder Fruchtsirup. Diese zuckerhaltigen Ausgangsstoffe zeichnen sich durch einen kleinen Ascheanteil von < 5 Gew.-%, insbesondere von < 2 Gew.-%, aus, was ebenfalls für die Eigenschaften der Formaktivkohle von Vorteil ist. Grundsätzlich können alle Kohlenhydrate, beispielsweise Monosaccharide (insbesondere Glucose, Fructose, Mannose, Galactose etc.) und/oder Disaccharide (insbesondere Saccharose, Maltose, Lactose, Cellobiose, Trehalose etc.) und/oder Tri-, Tetra-, Oligo- und Polysaccharide (insbesondere Stärke, Cellulose, Glykogen etc.) und/oder vorgelöste Stärke oder Cellulose, insbesondere in Form von wässrigen Lösungen, als Ausgangsstoffe eingesetzt werden. Auch Mischungen verschiedenster Zucker können eingesetzt werden.

Wird als Zuschlagstoff für die Umsetzung des wasserbasierten glucosehaltigen Ausgangsstoffes zu einem Bindemittel Phosphorsäure ausgewählt, ist es vorzugsweise vorgesehen und von Vorteil, dass nach dem Vermischen der Phosphorsäure mit dem wasserbasierten glucosehaltigen Ausgangsstoff das so erhältliche Bindemittel nicht neutralisiert wird. Wird dieses Bindemittel anschließend zur Herstellung von Formaktivkohle mit einem Kohlenstoffträger vermischt, kommt es zur Neutralisation saurer Gruppen des Bindemittels mit basischen Gruppen des Kohlenstoffträgers. Durch den Verzicht auf die Verfahrensstufe der Neutralisation wird der Herstellungsaufwand bei der Herstellung des Bindemittels deutlich vereinfacht. Darüber hinaus ist es auch möglich und ebenso von Vorteil, direkt ein Salz einer Phosphorsäure als Zuschlagstoff zu dem wasserbasierten glucosehaltigen Ausgangsstoff bei der Herstellung des Bindemittels einzusetzen.

Im Rahmen dieser Ausführungsform ist es bevorzugt, wenn der Zuschlagstoff der allgemeinen Formel (II)

$$[M_{m1}][H_{n1}P_{n2}O_{n3}]_{m1} \qquad (II)$$

entspricht,

- wobei M ein Proton ($H^+$) oder ein Kation, welches ausgewählt ist aus der Gruppe von Alkali-, Erdalkali-, Ammonium-, Calcium-, Magnesium- und Eisenionen, vorzugsweise aus Alkali-, Erdalkali- und Ammoniumionen, bezeichnet, wobei H Wasserstoff und P bzw. O Phosphor bzw. Sauerstoff bezeichnen,

- wobei m1 und m2 stöchiometrische Koeffizienten bezeichnen und ganze Zahlen mit m1 $\geq$ 1 und m2 $\geq$ 1 sind;

- wobei $[H_{n1}P_{n2}O_{n3}]$ ein Anion bezeichnet mit ganzzahligen stöchiometrischen Koeffizienten n1, n2 und n3 mit n1 > 0, n2 > 1; n3 > 2.

Als Zuschlagstoff für das kohlenhydrathaltige bzw. zuckerhaltige Bindemittel ist insbesondere Phosphorsäure ($H_3PO_4$) geeignet.

In Gegenwart von Phosphorsäuren wird der kohlenhydrathaltige Ausgangsstoff unter Bildung von Kohlenstoff dehydratisiert. Dieser Vorgang ist am Beispiel von Glucose in der nachfolgenden Gleichung illustriert:

$$C_{12}H_{22}O_{11} \rightarrow 12\ C + 11\ H_2O$$

Dabei entsteht eine Kohlenstoffmodifikation, die - im Vergleich zum zugesetzten Kohlenstoffträger (z. B. Holzkohle, Fruchtkerncarbonisat etc.) -nur langsam von Wasserdampf angegriffen wird.

Im Allgemeinen können Carbonate, Nitrate, Sulfate oder andere organische Salze als Vorstufen zur Bildung der Oberflächenoxide eingesetzt werden, die unter Einwirkung hoher Temperaturen größer 400 °C, bevorzugt jedoch bei Aktivierungstemperaturen von 500 bis 950 °C, Oxide bilden.

Der Zuschlagstoff kann weiterhin ausgewählt sein aus (Tri-)Ammoniumphosphat, (Di-)Ammoniumhydrogenphosphat, Ammoniumdihydrogenphosphat, (Tri-)Kaliumphosphat, (Di-)Kaliumhydrogenphosphat, Kaliumdihydrogenphosphat sowie deren Mischungen:

(Di-)Ammoniumhydrogenphosphat ist als Zuschlagstoff wegen der hohen Wasserlöslichkeit in einem wasserbasierten glucosehaltigen Ausgangsstoff besonders geeignet. Bei der Umsetzung des Zuschlagstoffes mit dem wasserbasierten kohlenhydrathaltigen bzw. glucosehaltigen Ausgangsstoff reagiert beispielsweise (Di-)Ammoniumhydrogenphosphat katalytisch mit dem Zucker des Bindemittels, wobei der Zucker in mehreren Reaktionsschritten aromatisiert wird. Der katalytische Effekt liegt insbesondere darin begründet, dass sich Phosphate an der OH-Gruppe des Zuckers unter Wasserabspaltung anlagern bzw. kondensieren und anschließend unter Ausbildung einer Doppelbindung im Zuckerring -letztendlich unter Aromatisierung bzw. Olefinisierung - abgespalten werden.

Wird die Aktivkohle mit Wasserdampf aktiviert, reagiert das aromatisierte Bindemittel während der Aktivierung der Formaktivkohle wesentlich schlechter mit Wasserdampf als der Kohlenstoffträger. Der Aromatisierungsprozess des Zuckers verläuft im Wesentlichen katalytisch, wobei der Ascheanteil in der Aktivkohle nicht oder nur unwesentlich ansteigt.

Beim Kohlenstoffträger handelt es sich vorzugsweise um Kohlenstoff aus nachwachsenden Rohstoffen, insbesondere um Holzkohle oder andere lignocellulosebasierte Naturstoffe. Grundsätzlich ist es jedoch auch möglich, fossile Kohlenstoffträger, insbesondere Braunkohle und/oder Braunkohlenkoks und/oder Steinkohle und/oder Mischungen aus nachwachsenden und fossilen Kohlenstoffträgern, mit dem Bindemittel zur Herstellung von Formaktivkohle zu mischen. Weiterhin können auch synthetische Polymere, beispielsweise auf Basis von Polyvinylbenzol oder ähnlicher, auch Heteroatome enthaltender synthetischer Polymere, als Kohlenstoffträger verwendet werden.

Die gemäß dieser Ausführungsform eingesetzte Formaktivkohle enthält die katalytisch aktiven Komponenten bzw. Dotierungsstoffe homogen verteilt in einer kohlenstoffhaltigen Matrix. Aufgrund der während des Herstellungsprozesses herrschenden hohen Temperaturen ist davon auszugehen, dass die Dotierungsstoffe teilweise und/oder vollständig chemisch verändert werden. Beispielsweise beträgt der Dissoziationsdruck von Kaliumcarbonat gemäß dem nachfolgenden Gleichgewicht bei 1000 °C etwa 5 torr:

$$K_2CO_3 \leftrightarrow K_2O + CO_2$$

Weiterhin ist bekannt, dass Kaliumcarbonat mit dem Kohlenstoffträger Oberflächenkomplexe bildet, die C-O-K-Fragmente enthalten. Ebenfalls wird in der Fachliteratur die Bildung von Interkalationsverbindungen postuliert, bei welchen insbesondere metallisches Kalium auf Zwischengitterplätzen einer Graphitgitterstruktur platziert ist. Röntgenstrukturanalysen von Aktivkohlen zeigen, dass Kohlenstoff nicht nur amorph, sondern auch in Form von sehr kleinen Kristallen, welche die normale Graphitgitterstruktur aufweisen, anzutreffen ist.

Es ist daher davon auszugehen - ohne sich auf diese Theorie festlegen zu wollen -, dass die Formaktivkohlen in den aus den Dotierungsstoffen entstandenen aktiven Zentren nicht mehr die ursprünglich eingesetzten Dotierungsstoffe, sondern zumindest teilweise Einheiten, insbesondere Cluster und Interkalationsverbindungen, mit anderer chemischer Struktur aufweisen. Es ist anzunehmen, dass sich die Interkalationsverbindungen nur auf die Graphitgitterstruktur beschränken.

Es ist weiterhin bekannt, dass sich Interkalationsverbindungen mit den Alkali-und Erdalkalimetallen bilden, die als sehr starke Reduktionsmittel agieren und zugleich auch an Wasserstoffspeicher- und Wasserstoffübertragungsreaktionen aktiv teilnehmen.

Für weitergehende diesbezügliche Einzelheiten zum Katalysator gemäß dieser Ausführungsform wird auf die DE 10 2004 033 561 A1 sowie die DE 10 2004 033 561 B4 verwiesen.

Gemäß einer weiteren, alternativen besonderen Ausführungsform der vorliegenden Erfindung kann als Katalysator eine Formaktivkohle eingesetzt werden, wie sie in der DE 10 2006 025 450 A1 bzw. in der zu derselben Patentfamilie gehörenden WO 2007/137856 A2 beschrieben ist, deren jeweiliger gesamter Offenbarungsgehalt hiermit durch Bezugnahme vollumfänglich eingeschlossen ist.

Gemäß dieser Ausführungsform kommt also als Katalysator eine Formaktivkohle zum Einsatz, welche herstellbar ist aus einer pressfähigen Masse, welche ein vermahlenes kohlenstoffhaltiges Material, ein Bindemittel und mindestens ein metallhaltiges Dotierungsreagenz enthält, und welche verpresst, getrocknet, carbonisiert und anschließend mittels eines Aktivierungsgases aktiviert wird, wobei ein erstes Dotierungsreagenz enthalten ist, wobei das erste Dotierungsreagenz ein Iodid der Alkali- oder Erdalkalimetalle ist, und wobei gegebenenfalls ein zweites Dotierungsreagenz der Formel $M^2_p(EO_q)_r$ enthalten ist, wobei $M^2$ ausgewählt ist aus Alkalimetallen und Erdalkalimetallen, E ein Element der 3. bis 7. Hauptgruppe des Periodensystems der Elemente ist und p, q und r jeweils ganze Zahlen $\geq 1$ sind; dabei kann das zweite Dotierungsreagenz insbesondere ausgewählt werden aus Hydroxiden und Carbonaten.

Ein weiterer Gegenstand der vorliegenden Erfindung - gemäß einem **zweiten** Aspekt der vorliegenden Erfindung - ist die Verwendung eines mit mindestens einem Metall versehenen Aktivkohlesubstrats als Katalysator gemäß Anspruch 1 für die

katalytische Umsetzung von Ethanol zu höheren Alkoholen und/oder Aldehyden sowie deren Gemischen, insbesondere $C_3$-$C_{30}$-Alkoholen und/oder $C_3$-$C_{30}$-Aldehyden sowie deren Gemischen.

Für weitergehende Einzelheiten zu diesem Erfindungsaspekt kann auf die vorstehenden Ausführungen zu dem ersten erfindungsgemäßen Aspekt verwiesen werden, welche entsprechend auch in Bezug auf diesen Erfindungsaspekt gelten.

Wiederum ein weiterer Gegenstand der vorliegenden Erfindung ist - gemäß einem **dritten** Aspekt der vorliegenden Erfindung - ein Verfahren zur Kettenverlängerung von mindestens eine Oxo- und/oder Hydroxyfunktion aufweisenden Kohlenstoffverbindungen durch katalytische Umsetzung in Gegenwart mindestens eines Katalysators, gemäß Anspruch 14 wobei der Katalysator ein Aktivkohlesubstrat, welches mit mindestens einem Metall versehen ist, umfasst.

Das erfindungsgemäße Verfahren kann folglich nicht nur zur Herstellung höherer Alkohole bzw. Aldehyde aus Ethanol enthaltenen Eduktgemischen verwendet werden; vielmehr kann das Verfahren nach der Erfindung auch allgemein zur Kettenverlängerung von Oxo- und/oder Hydroxyfunktionen aufweisenden Kohlenstoffverbindungen verwendet werden. Insbesondere primäre und sekundäre Alkohole sowie Aldehyde und Ketone eignen sich für diese Art der Verfahrensführung, wobei lediglich sichergestellt werden muss, dass mindestens eine Eduktkomponente mindestens ein Wasserstoffatom an einem Kohlenstoffatom in alpha-Position bzw. vicinaler Position zur funktionellen Gruppe aufweist. Das heißt, Methanol und Formaldehyd bzw. deren Mischungen sind als alleinige Eduktkomponenten nicht geeignet.

Von dieser Ausnahme abgesehen, lässt sich mit dem erfindungsgemäßen Verfahren eine Vielzahl von Oxound Hydroxyfunktionen aufweisenden Kohlenstoffverbindungen, insbesondere primäre und sekundäre Alkohole sowie Aldehyde und Ketone, umsetzen. Insbesondere lassen sich auch Verbindungen mit mehr als drei oder vier Kohlenstoffatomen als Edukte einsetzen und in Produkte mit einer noch höheren Kettenlänge umwandeln. Limitiert wird dieser Prozess der Kettenverlängerung allenfalls durch die Bedingung, dass sowohl Produkte als auch Edukte nicht im Reaktor kondensieren sollten, d. h. es sollte insbesondere nicht zur Ausbildung einer flüssigen Phase im Reaktor kommen, da andernfalls der Katalysator geschädigt werden könnte.

Für weitergehende Einzelheiten zu diesem Erfindungsaspekt kann auf die Ausführungen zu den anderen Erfindungsaspekten verwiesen werden, welche entsprechend gelten und/oder Hydroxyfunktion aufweisenden Kohlenstoffverbindungen.

Für weitergehende diesbezügliche Einzelheiten zu diesem Erfindungsaspekt kann Bezug genommen werden auf die obigen Ausführungen zu den weiteren Erfindungsaspekten, welche in Bezug auf diese erfindungsgemäße Verwendung entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die vorliegende Erfindung wird anhand der nachfolgenden Ausführungsbeispiele veranschaulicht.

### Ausführungsbeispiele

Beispiel 1 (Druckloses System)

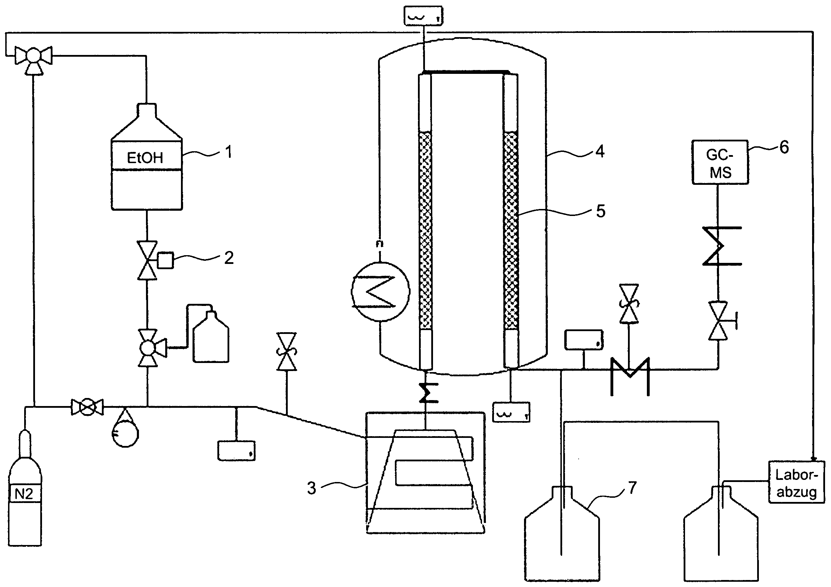

Erfindungsgemäß hergestellte Pellets aus Formaktivkohle werden mechanisch zerkleinert und die Bruchstücke (1-2 mm Fraktion) in einen Edelstahlreaktor eingefüllt. Der Reaktor hat einen inneren Durchmesser von 9 mm und eine Länge von 135 mm. Die Masse des eingefüllten Katalysators beträgt 10 g bzw. 45 g. Eine schematische Darstellung des verwendeten Versuchsaufbaus ist in der einzigen Figur abgebildet.

Als Ausgangsstoff bzw. Reaktand kommt sowohl reines Ethanol als auch wässriges Ethanol in Betracht. Ebenso sind Mischungen verschiedener Alkohole und Aldehyde möglich.

Die Reaktanden befinden sich in einem Druckbehälter 1 und werden über einen kalibrierten Massendurchflussregler 2 dosiert. Vor dem Eintritt in den Verdampfer kann optional Inertgas, in der Regel Stickstoff, hinzugegeben werden. Der Volumenstrom wird über ein Nadelventil mit einem Schwebkörperdurchflussmesser eingestellt und liegt typischerweise zwischen 0 und 40 1 $N_2$/h. In einem elektrisch beheizten Rohr 3 werden die Reaktanden verdampft und auf bis zu 380 °C erhitzt. Das Inertgas wird ebenfalls auf bis zu 380 °C erwärmt. Das gasförmige Gemisch strömt vom Verdampfer in den Reaktor 4, in dem an der Oberfläche des Katalysators 5 die Reaktion abläuft. Die Temperaturen im Reaktor liegen zwischen 350 und 425 °C. Typische Kontaktzeiten betragen zwischen 0,01 und 30 Sekunden. Nach Durchlauf des Reaktors wird das Produktgemisch in eine oder mehrere Kühlfallen 7 kondensiert und der bei Raumtemperatur nicht kondensierbare Anteil gasförmig abgeführt. Über eine beheizte Leitung ist eine direkte Probennahme aus dem Produktgasstrom möglich.

Die Zusammensetzung der flüssigen Produkte wird mittels HPLC (HPLC 1200 mit RI-Detektor und einer Rezex ROA, 300 x 7,8 mm Säule von Phenomenex der Firma Agilent) und der Wassergehalt der Probe mittels Karl-Fischer-Titration bestimmt. Weitere Produkte werden mittels GC/MS (GC/MS+FID 6890N/5975 mit einer DB-FFAP, 30m x 0,25mm x 0,25 µm Säule der Firma Agilent) bestimmt.

Ausgewählte Versuchsergebnisse finden sich in den angeführten Tabellen (Versuche 1 bis 4, 8 und 14 bis 17). Neben den dort aufgeführten Verbindungen findet sich noch hauptsächlich Wasser aus der Reaktion im Produkt. Weiterhin finden sich je nach Prozessbedingungen ebenfalls Xylol und Alkylbenzole sowie Ethyloktanol und Dodekanol im Produkt.

Neben der flüssigen Phase wird auch die Zusammensetzung der entstehenden gasförmigen Produkte bestimmt (die Analysenergebnisse sind nicht explizit aufgeführt). Die Analysen zeigen, dass hier Wasserstoff, Ethan und Kohlenstoffdioxid die Hauptanteile (in Mol-%) bilden. Weitere Komponenten sind Spuren von Alkanen und Alkenen verschiedenster Kettenlängen. Insgesamt entsteht, speziell bei Temperaturen unter 380°C, jedoch nur ein sehr geringer Anteil gasförmiger Produkte (< 5 Gew.-%). Mit steigender Temperatur oder Verweilzeit kann der Anteil allerdings auf bis zu 20 Gew.-% ansteigen.

Neben der Untersuchung zur Umsetzung reinen oder wässrigen Ethanols können mit dem Verfahren auch Mischungen von Alkoholen bzw. Aldehyden umgesetzt werden. Ein Versuch mit einem Methanol/Ethanol-Eduktgemisch ist exemplarisch in Tabelle 2 als Versuch 7 aufgeführt. Im Vergleich zur Verwendung reinen Ethanols steigt bei Verwendung eines Methanol/Ethanol-Gemisches der Anteil verzweigter Verbindungen sowie auch der Anteil an Verbindungen mit einer ungeraden Anzahl C-Atome deutlich an.

Beispiel 2 (System unter erhöhtem Druck)

Um auch Versuche unter erhöhtem Druck durchzuführen, wurde eine zweite Anlage genutzt, in welcher das Ethanol mittels einer HPLC-Pumpe aus einer Vorlage in einen mit Öl auf 300 °C beheizten Verdampfer gefördert wird. Die Reaktion läuft in einem auf Reaktionstemperatur elektrisch beheizten Reaktor mit einem Volumen von 120 ml an 45 g des beschriebenen Katalysators ab. Das Produkt wird nach der Reaktion in zwei wassergekühlten Wärmetauschern kondensiert. Mit dieser Anlage werden Versuche mit einem Druck bis zu 21 bar (absolut) durchgeführt. Der Ethanolmassenstrom beträgt zwischen 45 und 90 g/h. Die Ergebnisse finden sich in den Tabellen als Versuche 5 und 6 sowie 9 bis 13.

Eine Druckerhöhung führt vor allem zu einer deutlichen Verringerung der Konzentration der Aldehyde im Produkt. Ebenso steigt der Anteil der höheren Alkohole im Vergleich zur drucklosen Prozessführung bereits bei geringeren Temperaturen deutlich an.

Eingesetzte Katalysatorsysteme

In den vorangehenden Ausführungsbeispielen kommen als Katalysatoren alkali- und erdalkalidotierte, mittels Phosphat basisch eingestellte Aktivkohlesubstrate zum Einsatz, wie sie gemäß DE 10 2004 033 561 A1 erhalten werden. Die eingesetzten Katalysatorsysteme "Kat1", Kat2" und "Kat3" sind nachfolgend näher chemisch charakterisiert: Chemische Zusammensetzung der eingesetzten Katalysatoren, bezogen auf Asche:

| Kat1: | Aschegehalt | 24,4 | Gew.-% |
|---|---|---|---|
| | Natrium | 7,28 | g/kg |
| | Kalium | 238 | g/kg |
| | Magnesium | 11,7 | g/kg |
| | Phosphor | 19,2 | g/kg |
| | Calcium | 167 | g/kg |
| | Summe | 443,18 | g/kg |

| Kat2: | Aschegehalt | 18,1 | Gew.-% |
|---|---|---|---|
| | Natrium | 10,7 | g/kg |

(fortgesetzt)

| | | | |
|---|---|---|---|
| Kalium | 360 | g/kg |
| Magnesium | 13,9 | g/kg |
| Phosphor | 36,5 | g/kg |
| Calcium | 110 | g/kg |
| Summe | 531,1 | g/kg |

| | | | | |
|---|---|---|---|---|
| Kat3: | Aschegehalt | 11,0 | Gew.-% |
| | Natrium | 22,2 | g/kg |
| | Kalium | 307 | g/kg |
| | Magnesium | 11,8 | g/kg |
| | Phosphor | 5,6 | g/kg |
| | Calcium | 130 | g/kg |
| | Summe | 476,6 | g/kg |

Molare chemische Zusammensetzung der getesteten Katalysatoren:

| Kat1 | Na | K | Mg | Ca | P | 0,5 | Na/P | 0,8 | Mg/P |
|------|-----|-----|-----|-----|-----|------|------|------|------|
|      | 0,3 | 6,1 | 0,5 | 4,2 | 0,6 | 9,8  | K/P  | 6,7  | Ca/P |
| Kat2 | Na | K | Mg | Ca | P | 0,4 | Na/P | 0,5 | Mg/P |
|      | 0,5 | 9,2 | 0,6 | 2,7 | 1,2 | 7,8  | K/P  | 2,3  | Ca/P |
| Kat3 | Na | K | Mg | Ca | P | 5,3 | Na/P | 2,7 | Mg/P |
|      | 1,0 | 7,9 | 0,5 | 3,2 | 0,2 | 43,5 | K/P  | 18,0 | Ca/P |

**Tabelle 1:** Produktzusammensetzungen von Versuchen mit Ethanol als Ausgangsmaterial bei unterschiedlichen Reaktionsbedingungen; es wurden 10 g (druckloses System; 1 bar) bzw. 45 g Katalysator (System unter erhöhtem Druck) eingesetzt

| Nr. | Ethanol [g/l] | Butanol [g/l] | Hexanol [g/l] | Oktanol [g/l] | Ethanal [g/l] | Butanal [g/l] | Hexanal [g/l] | Alkohole ges. [g/l] | Aldehyde ges. [g/l] | T [°C] | m [g/h] | $N_2$ [1/h] | p [bar] |
|---|---|---|---|---|---|---|---|---|---|---|---|---|---|
| 1 | 495 | 68 | 8,1 | 1,4 | 39,8 | 11,5 | 2,4 | 78 | 53,7 | 405 | 20 | 1 | 1 |
| 2 | 626 | 55,9 | 6,8 | 0 | 14 | 4 | 0 | 63 | 18,0 | 385 | 15 | 25 | 1 |
| 3 | 281 | 72,0 | 13,6 | 1,4 | 27,7 | 20,0 | 8,7 | 87 | 56,4 | 425 | 5 | 4 | 1 |
| 4 | 549 | 77,7 | 13,7 | 3 | 11,5 | 11,5 | 3,7 | 94 | 26,7 | 350 | 2 | 4 | 1 |
| 5 | 512 | 80,5 | 19,9 | 5,6 | 8,0 | 1 | 0 | 106 | 9,0 | 360 | 60 | 0 | 11 |
| 6 | 482 | 91,3 | 23,8 | 6,3 | 5,9 | 6,3 | 1,3 | 121 | 13,4 | 380,0 | 60,0 | 0 | 21 |

**Tabelle 2:** Produktzusammensetzung eines Versuchs mit einer Methanol/Ethanol-Mischung (5:1) als Ausgangsmaterial unter Normaldruck (1 bar) und 10 g Katalysator

| Nr. | Methanol [g/l] | Ethanol [g/l] | Propanol [g/l] | Iso-butanol [g/l] | Butanol [g/l] | Hexanol [g/l] | Oktanol [g/l] | Ethanal [g/l] |
|---|---|---|---|---|---|---|---|---|
| 7 | 344 | 141 | 82 | 51 | 17,9 | 1,1 | 0 | 7,4 |
|  | Butanal [g/l] | Hexanal [g/l] | Alkohole ges. [g/l] | Aldehyde ges. [g/l] | T[°C] | m [g/h] | N [l/h] |  |
|  | 0 | 0,0 | 152,2 | 7,4 | 400 | 8 | 30 |  |

**Tabelle 3**: Versuchsbedingungen und stoffmengenbezogene Umsätze weiterer Versuche (Nr. 8 bis 17) mit Ethanol als Ausgangsverbindung

| Nr. | Temperatur [°C] | Druck [bar] | Massenstrom Edukt [g/h] | Katalysatormasse [g] | Katalysatorvolumen [l] | Stickstoffvolumenstrom [l/h] | Raum-Zeit-Ausbeute [g/(h·l)] | Umsatz [%] |
|---|---|---|---|---|---|---|---|---|
| 8 | 360 | 1 | 11,4 | 45 | 0,1 | 0 | 74 | 65 |
| 9 | 380 | 21 | 90 | 45 | 0,1 | 0 | 398 | 44 |
| 10 | 380 | 21 | 45 | 45 | 0,1 | 0 | 279 | 62 |
| 11 | 380 | 21 | 60 | 45 | 0,1 | 0 | 371 | 62 |
| 12 | 380 | 11 | 90 | 45 | 0,1 | 0 | 465 | 52 |
| 13 | 360 | 11 | 60 | 45 | 0,1 | 0 | 337 | 56 |
| 14 | 405 | 1 | 20 | 10 | 0,015 | 1 | 695 | 52 |
| 15 | 385 | 1 | 15 | 10 | 0,015 | 25 | 447 | 45 |
| 16 | 425 | 1 | 5 | 10 | 0,015 | 4 | 276 | 83 |
| 17 | 350 | 1 | 2 | 10 | 0,015 | 4 | 55 | 41 |

**Tabelle 4:** Produktverteilung und stoffmengenbezogene Selektivitäten [%] der Versuche 8 bis 17 mit Ethanol als Ausgangsverbindung

| Nr. | Acetaldehyd | Butanal | Hexanal | Oktanal | Butanol | Hexanol | Oktanol | Xylol | Ethylbutanol | Alkylbenzol | Ethylhexanol | Decanol | Summe |
|---|---|---|---|---|---|---|---|---|---|---|---|---|---|
| 8 | 4,9 | 4,9 | 1,7 | 0,4 | 28,9 | 7,3 | 1,8 | 1,4 | 3,0 | 0,7 | 1,7 | 0,7 | 57,4 |
| 9 | 3,2 | 0,0 | 0,0 | 0,0 | 25,7 | 4,3 | 0,7 | n.b. | n.b. | n.b. | n.b. | n.b. | 33,8 |
| 10 | 1,0 | 1,2 | 0,2 | 0,0 | 18,5 | 5,2 | 1,5 | 1,4 | 2,5 | 0,7 | 1,5 | 0,7 | 34,6 |
| 11 | 1,7 | 0,0 | 0,3 | 0,0 | 22,6 | 5,1 | 1,1 | 2,1 | 2,8 | 0,9 | 1,3 | 0,5 | 38,3 |
| 12 | 3,4 | 0,0 | 0,0 | 0,0 | 15,5 | 2,2 | 0,0 | n.b. | n.b. | n.b. | n.b. | n.b. | 21,0 |
| 13 | 1,8 | 2,1 | 0,6 | 0,0 | 26,1 | 7,9 | 2,4 | 1,2 | 3,1 | 0,7 | 2,0 | 1,3 | 49,1 |
| 14 | 7,7 | 2,7 | 0,6 | 0,4 | 15,7 | 2,0 | 0,4 | n.b. | n.b. | n.b. | n.b. | n.b. | 29,5 |
| 15 | 2,9 | 1,0 | 0,0 | 0,4 | 13,8 | 1,8 | 0,0 | n.b. | n.b. | n.b. | n.b. | n.b. | 19,9 |
| 16 | 2,2 | 2,0 | 0,9 | 0,0 | 6,9 | 1,4 | 0,2 | n.b. | n.b. | n.b. | n.b. | n.b. | 13,6 |
| 17 | 6,0 | 0,8 | 0,0 | 0,0 | 20,9 | 2,6 | 0,5 | n.b. | n.b. | n.b. | n.b. | n.b. | 30,8 |

n.b. = nicht bestimmt

**Tabelle 5:** Stoffmengenbezogene Ausbeuten [%] der Versuche 8 bis 17 mit Ethanol als Ausgangsverbindung

| Nr. | Acetaldehyd | Butanal | Hexanal | Oktanal | Butanol | Hexanol | Oktanol | Xylol | Ethylbutanol | Alkylbenzol | Ethylhexanol | Decanol | Summe |
|---|---|---|---|---|---|---|---|---|---|---|---|---|---|
| 8 | 3,2 | 3,2 | 1,1 | 0,2 | 18,8 | 4,7 | 1,2 | 0,9 | 2,0 | 0,4 | 1,1 | 0,5 | 37,4 |
| 9 | 1,4 | 0,0 | 0,0 | 0,0 | 11,4 | 1,9 | 0,3 | n.b. | n.b. | n.b. | n.b. | n.b. | 15,0 |
| 10 | 0,6 | 0,8 | 0,1 | 0,0 | 11,5 | 3,3 | 0,9 | 0,9 | 1,6 | 0,5 | 0,9 | 0,5 | 21,5 |
| 11 | 1,0 | 0,0 | 0,2 | 0,0 | 14,0 | 3,2 | 0,7 | 1,3 | 1,7 | 0,5 | 0,8 | 0,3 | 23,7 |
| 12 | 1,8 | 0,0 | 0,0 | 0,0 | 8,0 | 1,1 | 0,0 | n.b. | n.b. | n.b. | n.b. | n.b. | 10,9 |
| 13 | 1,0 | 1,2 | 0,3 | 0,0 | 14,6 | 4,5 | 1,3 | 0,7 | 1,7 | 0,4 | 1,1 | 0,7 | 27,5 |
| 14 | 4,0 | 1,4 | 0,3 | 0,2 | 8,2 | 1,1 | 0,2 | n.b. | n.b. | n.b. | n.b. | n.b. | 15,4 |
| 15 | 1,3 | 0,5 | 0,0 | 0,2 | 6,1 | 0,8 | 0,0 | n.b. | n.b. | n.b. | n.b. | n.b. | 8,9 |
| 16 | 1,8 | 1,6 | 0,8 | 0,0 | 5,7 | 1,2 | 0,1 | n.b. | n.b. | n.b. | n.b. | n.b. | 11,2 |
| 17 | 2,5 | 0,3 | 0,0 | 0,0 | 8,7 | 1,1 | 0,2 | n.b. | n.b. | n.b. | n.b. | n.b. | 12,8 |

n.b. = nicht bestimmt

**Patentansprüche**

1. Verfahren zur Herstellung höherer Alkohole und/oder Aldehyde sowie deren Gemischen durch katalytische Umsetzung von Ethanol, wobei die Umsetzung in Gegenwart mindestens eines Katalysators erfolgt, wobei der Katalysator ein Aktivkohlesubstrat, welches mit mindestens einem Metall versehen ist, umfasst, wobei der Katalysator mindestens ein einwertiges Metall $M^I$ in Form eines Alkalimetalls und mindestens ein zweiwertiges Metall $M^{II}$ in Form von Calcium und/oder Magnesium aufweist und wobei der Katalysator Phosphor in Form von Phosphaten enthält, wobei die folgenden molaren Verhältnisse gelten:

   (i) $0,5 \leq M^I/M^{II} \leq 5$; und
   (ii) $2 \leq M^{II}/P \leq 30$; und
   (iii) $1 \leq M^I/P \leq 60$,

   wobei der Katalysator die folgenden Mengenanteile der nachfolgend genannten Komponenten umfasst, jeweils bezogen auf den Katalysator:

   (i) $M^I$: 0,1 bis 20 Gew.-%; und
   (ii) $M^{II}$ in Form von Calcium und/oder Magnesium: 0,1 bis 20 Gew.-%; und
   (iii) P in Form von Phosphat, berechnet als Phosphor P: 0,01 bis 5 Gew.-%.

2. Verfahren nach Anspruch 1, wobei die höheren Alkohole und/oder Aldehyde ausgewählt sind aus linearen und/oder verzweigten Alkoholen und/oder Aldehyden und/oder wobei ein Butanol(e) enthaltendes Produktgemisch resultiert.

3. Verfahren nach einem der vorangehenden Ansprüche, wobei die Umsetzung in der Gasphase erfolgt und/oder wobei die Umsetzung oberhalb der Siedetemperaturen der Edukte und/oder Produkte erfolgt; und/oder wobei die Umsetzung bei Temperaturen im Bereich von 150 °C bis 600 °C durchgeführt wird.

4. Verfahren nach einem der vorangehenden Ansprüche, wobei die Umsetzung bei reduziertem Druck, Normaldruck oder bei Überdruck durchgeführt wird.

5. Verfahren nach einem der vorangehenden Ansprüche, wobei die Umsetzung mit Reaktionsdauern und/oder Kontaktzeiten im Bereich von 0,001 bis 120 Sekunden durchgeführt wird.

6. Verfahren nach einem der vorangehenden Ansprüche, wobei der Katalysator und/oder das Aktivkohlesubstrat basisch ausgerüstet und/oder ausgebildet ist und/oder wobei der Katalysator und/oder das Aktivkohlesubstrat mindestens eine basische funktionelle Gruppe und/oder mindestens eine basische chemische Verbindung aufweist.

7. Verfahren nach Anspruch 6, wobei die basische Ausrüstung durch (i) Hydroxide; (ii) Oxide; (iii) Salze anorganischer Säuren; (iv) Salze organischer Säuren; und/oder (v) Alkoholate bereitgestellt wird; und/oder wobei die basische Ausrüstung bei der Herstellung des Katalysators oder aber nachträglich erfolgt.

8. Verfahren nach einem der vorangehenden Ansprüche, wobei der Katalysator und/oder das Aktivkohlesubstrat eine spezifische Oberfläche (BET) im Bereich von 450 bis 3.000 $m^2$/g aufweist.

9. Verfahren nach einem der vorangehenden Ansprüche, wobei der Katalysator und/oder das Aktivkohlesubstrat ein Mikroporenvolumen nach Gurvich im Bereich von 0,1 bis 3,0 ml/g aufweist.

10. Verfahren nach einem der vorangehenden Ansprüche, wobei der Katalysator und/oder das Aktivkohlesubstrat mindestens eine funktionelle Gruppe umfasst, ausgewählt aus Carbonyl-, Carboxylat-, Hydroxyl-, Oxid-, Ether-, Ester-, Lacton-, Phenol- und/oder Chinongruppen.

11. Verfahren nach einem der vorangehenden Ansprüche, wobei als Katalysator eine basisch ausgerüstete und/oder eingestellte Aktivkohle, welche mindestens eine Alkali-und Calcium- und/oder Magnesiumdotierung aufweist, eingesetzt wird.

12. Verfahren nach einem der vorangehenden Ansprüche, wobei der Katalysator als einwertiges Metall $M^I$ Natrium und/oder Kalium aufweist.

13. Verfahren nach Anspruch 12, wobei die folgenden molaren Verhältnisse gelten:

(i) $2 \leq M^I/M^{II} \leq 3$; und/oder
(ii) $2 \leq M^{II}/P \leq 8$; und/oder
(iii) $5 \leq M^I/P \leq 10$; und/oder
(iv) $1 \leq K/Na \leq 20$; und/oder
(v) $1 \leq Ca/Mg \leq 10$.

14. Verfahren zur Kettenverlängerung von mindestens eine Oxo- und/oder Hydroxyfunktion aufweisenden Kohlenstoffverbindungen durch katalytische Umsetzung in Gegenwart mindestens eines Katalysators, wobei der Katalysator ein Aktivkohlesubstrat, welches mit mindestens einem Metall versehen ist, umfasst, wobei der Katalysator mindestens ein einwertiges Metall $M^I$ in Form eines Alkalimetalls und mindestens ein zweiwertiges Metall $M^{II}$ in Form von Calcium und/oder Magnesium aufweist und wobei der Katalysator Phosphor in Form von Phosphaten enthält, wobei die folgenden molaren Verhältnisse gelten:

(i) $0{,}5 \leq M^I/M^{II} \leq 5$; und
(ii) $2 \leq M^{II}/P \leq 30$; und
(iii) $1 \leq M^I/P \leq 60$,

wobei der Katalysator die folgenden Mengenanteile der nachfolgend genannten Komponenten umfasst, jeweils bezogen auf den Katalysator:

(i) $M^I$: 0,1 bis 20 Gew.-%; und
(ii) $M^{II}$ in Form von Calcium und/oder Magnesium: 0,1 bis 20 Gew.-%; und
(iii) P in Form von Phosphat, berechnet als Phosphor P: 0,01 bis 5 Gew.-%.

**Claims**

1. A process for preparing higher alcohols and/or aldehydes and also mixtures thereof by catalytic reaction of ethanol, wherein the reaction is carried out in the presence of at least one catalyst, wherein the catalyst comprises an activated carbon substrate which is provided with at least one metal, wherein the catalyst has at least one monovalent metal $M^I$, in the form of an alkali metal, and at least one divalent metal $M^{II}$, in the form of calcium and/or magnesium, and wherein the catalyst contains phosphorus in the form of phosphates,
wherein the following molar ratios apply:

(i) $0.5 \cdot M^I/M^{II} \cdot 5$; and
(ii) $2 \cdot M^{II}/P \cdot 30$; and
(iii) $1 \cdot M^I/P \cdot 60$

wherein the catalyst comprises the following proportions of the following components, in each case based on the catalyst:

(i) $M^I$ : 0.1 to 20 % by weight; and
(ii) $M^{II}$ in the form of calcium and/or magnesium: 0.1 to 20 % by weight; and
(iii) P in the form of phosphate, calculated as phosphorus P: 0.01 to 5 % by weight.

2. The process as claimed in claim 1,
wherein the higher alcohols and/or aldehydes are selected from linear and/or branched alcohols and/or aldehydes and/or wherein a butanol(s) containing product mixture results.

3. The process as claimed in any of the preceding claims,
wherein the reaction is carried out in the gas phase and/or the reaction is carried out at above the boiling points of the starting materials and/or products; and/or wherein the reaction is carried at temperatures in the range from 150°C to 600°C.

**4.** The process as claimed in any of the preceding claims,
wherein the reaction is carried out at reduced pressure, atmospheric pressure or superatmospheric pressure.

**5.** The process as claimed in any of the preceding claims,
wherein the reaction is carried out using reaction times and/or contact times in the range from 0.001 to 120 seconds.

**6.** The process as claimed in any of the preceding claims,
wherein the catalyst and/or the activated carbon substrate is equipped and/or configured so as to be basic and/or the catalyst and/or the activated carbon substrate have/has at least one basic functional group and/or at least one basic chemical compound.

**7.** The process as claimed in claim 6,
wherein the basic equipping is provided by (i) hydroxides; (ii) oxides; (iii) salts of inorganic acids; (iv) salts of organic acids; and/or (v) alkoxides; and/or
wherein the basic equipping can be effected during the production of the catalyst or else subsequently.

**8.** The process as claimed in any of the preceding claims,
wherein the catalyst and/or the activated carbon substrate has a specific surface area (BET) in the range from 450 to 3000 $m^2/g$.

**9.** The process as claimed in any of the preceding claims,
wherein the catalyst and/or the activated carbon substrate has a micropore volume determined by the Gurvich method, in the range from 0.1 to 3.0 ml/g.

**10.** The process as claimed in any of the preceding claims, wherein the catalyst and/or the activated carbon substrate comprises at least one functional group selected from carbonyl, carboxylate, hydroxyl, oxide, ether, ester, lactone, phenol and/or quinone groups.

**11.** The process as claimed in any of the preceding claims,
wherein an activated carbon which is equipped and/or configured so as to be basic and has at least a alkali metal and calcium and/or magnesium doping is used as catalyst.

**12.** The process as claimed in any of the preceding claims,
wherein the catalyst comprises as monovalent metal $M^I$ sodium and/or potassium.

**13.** The process as claimed in claim 12, wherein the following molar ratios apply:

(i) $2 \cdot M^I/M^{II} \cdot 3$; and/or
(ii) $2 \cdot M^{II}/P \cdot 8$; and/or
(iii) $5 \cdot M^I/P \cdot 10$; and/or
(iv) $1 \cdot K/Na \cdot 20$, and/or
(v) $1 \cdot Ca/Mg \cdot 10$.

**14.** A process for the chain extension of carbon compounds having at least one oxo and/or hydroxy function by catalytic reaction in the presence of at least one catalyst, wherein the catalyst is an activated carbon substrate provided with at least one metal,
wherein the catalyst has at least one monovalent metal $M^I$, in the form of an alkali metal, and at least one divalent metal $M^{II}$, in the form of calcium and/or magnesium, and wherein the catalyst contains phosphorus in the form of phosphates,
wherein the following molar ratios apply:

(i) $0.5 \cdot M^I/M^{II} \cdot 5$; and
(ii) $2 \cdot M^{II}/P \cdot 30$; and
(iii) $1 \cdot M^I/P \cdot 60$

wherein the catalyst comprises the following proportions of the following components, in each case based on the catalyst:

(i) $M^I$: 0.1 to 20 % by weight; and
(ii) $M^{II}$ in the form of calcium and/or magnesium: 0.1 to 20 % by weight; and
(iii) P in the form of phosphate, calculated as phosphorus P: 0.01 to 5 % by weight.

**Revendications**

1. Procédé de fabrication d'alcools et/ou d'aldéhydes supérieurs, ainsi que leurs mélanges, par réaction catalytique d'éthanol, la réaction ayant lieu en présence d'au moins un catalyseur, le catalyseur comprenant un substrat en charbon actif, qui est muni d'au moins un métal, le catalyseur comprenant au moins un métal monovalent $M^I$ sous la forme d'un métal alcalin et au moins un métal bivalent $M^{II}$ sous la forme de calcium et/ou de magnésium, et le catalyseur contenant du phosphore sous la forme de phosphates, les rapports molaires suivants étant vérifiés :

   (i) $0,5 \leq M^I/M^{II} \leq 5$ ; et
   (ii) $2 \leq M^{II}/P \leq 30$ ; et
   (iii) $1 \leq M^I/P \leq 60$,

   le catalyseur comprenant les proportions suivantes des composants mentionnés ci-dessous, à chaque fois par rapport au catalyseur :

   (i) $M^I$ : 0,1 à 20 % en poids, et
   (ii) $M^{II}$ sous la forme de calcium et/ou de magnésium : 0,1 à 20 % en poids ; et
   (iii) P sous la forme de phosphate, calculé en tant que phosphore P : 0,01 à 5 % en poids.

2. Procédé selon la revendication 1, dans lequel les alcools et/ou aldéhydes supérieurs sont choisis parmi les alcools et/ou aldéhydes linéaires et/ou ramifiés et/ou dans lequel un mélange de produits contenant un ou plusieurs butanols est obtenu.

3. Procédé selon l'une quelconque des revendications précédentes,
   dans lequel la réaction a lieu en phase gazeuse et/ou dans lequel la réaction a lieu au-dessus des températures d'ébullition des réactifs et/ou des produits ; et/ou
   dans lequel la réaction est réalisée à des températures dans la plage s'étendant de 150 °C à 600 °C.

4. Procédé selon l'une quelconque des revendications précédentes, dans lequel la réaction est réalisée à pression réduite, à pression normale ou à une surpression.

5. Procédé selon l'une quelconque des revendications précédentes, dans lequel la réaction est réalisée avec des durées de réaction et/ou des temps de contact dans la plage s'étendant de 0,001 à 120 secondes.

6. Procédé selon l'une quelconque des revendications précédentes, dans lequel le catalyseur et/ou le substrat en charbon actif sont apprêtés et/ou configurés sous forme basique et/ou dans lequel le catalyseur et/ou le substrat en charbon actif comprennent au moins un groupe fonctionnel basique et/ou au moins un composé chimique basique.

7. Procédé selon la revendication 6,
   dans lequel l'apprêt basique est fourni par (i) des hydroxydes ; (ii) des oxydes ; (iii) des sels d'acides inorganiques ; (iv) des sels d'acides organiques ; et/ou (v) des alcoolates ;
   et/ou
   dans lequel l'apprêt basique a lieu lors de la fabrication du catalyseur ou ultérieurement.

8. Procédé selon l'une quelconque des revendications précédentes, dans lequel le catalyseur et/ou le substrat en charbon actif présentent une surface spécifique (BET) dans la plage s'étendant de 450 à 3 000 $m^2$/g.

9. Procédé selon l'une quelconque des revendications précédentes, dans lequel le catalyseur et/ou le substrat en charbon actif présentent un volume de micropores selon Gurvich dans la plage allant de 0,1 à 3,0 ml/g.

10. Procédé selon l'une quelconque des revendications précédentes, dans lequel le catalyseur et/ou le substrat en charbon actif comprennent au moins un groupe fonctionnel, choisi parmi les groupes carbonyle, carboxylate, hydroxyle, oxyde, éther, ester, lactone, phénol et/ou quinone.

**11.** Procédé selon l'une quelconque des revendications précédentes, dans lequel un charbon actif apprêté et/ou ajusté sous forme basique, qui comprend au moins un dopage par un alcali et du calcium et/ou du magnésium, est utilisé en tant que catalyseur.

**12.** Procédé selon l'une quelconque des revendications précédentes, dans lequel le catalyseur comprend en tant que métal monovalent $M^I$ du sodium et/ou du potassium.

**13.** Procédé selon la revendication 12, dans lequel les rapports molaires suivants sont vérifiés :

(i) $\leq M^I/M^{II} \leq 3$; et/ou
(ii) $2 \leq M^{II}/P \leq 8$; et/ou
(iii) $5 \leq M^I/P \leq 10$; et/ou
(iv) $1 \leq K/Na \leq 20$ ; et/ou
(v) $1 \leq Ca/Mg \leq 10$.

**14.** Procédé d'allongement de chaînes de composés carbonés comprenant au moins une fonction oxo et/ou hydroxy par réaction catalytique en présence d'au moins un catalyseur, le catalyseur comprenant un substrat en charbon actif, qui est muni d'au moins un métal, le catalyseur comprenant au moins un métal monovalent $M^I$ sous la forme d'un métal alcalin et au moins un métal bivalent $M^{II}$ sous la forme de calcium et/ou de magnésium, et le catalyseur contenant du phosphore sous la forme de phosphates, les rapports molaires suivants étant vérifiées :

(i) $0,5 \leq M^I/M^{II} \leq 5$ ; et
(ii) $2 \leq M^{II}/P \leq 30$ ; et
(iii) $1 \leq M^I/P \leq 60$,

le catalyseur comprenant les proportions suivantes des composants mentionnés ci-dessous, à chaque fois par rapport au catalyseur :

(i) $M^I$ : 0,1 à 20 % en poids, et
(ii) $M^{II}$ sous la forme de calcium et/ou de magnésium : 0,1 à 20 % en poids ; et
(iii) P sous la forme de phosphate, calculé en tant que phosphore P : 0,01 à 5 % en poids.

**IN DER BESCHREIBUNG AUFGEFÜHRTE DOKUMENTE**

**In der Beschreibung aufgeführte Patentdokumente**

- EP 1829851 A1
- WO 2009026518 A1
- WO 2009026483 A1
- WO 2009026501 A1
- WO 2009026506 A1
- WO 2009026523 A1
- WO 2009097310 A1

- WO 2009026510 A1
- WO 2009097312 A1
- EP 1052234 A
- DE 102004033561 A1
- DE 102004033561 B4
- DE 102006025450 A1
- WO 2007137856 A2

**In der Beschreibung aufgeführte Nicht-Patentliteratur**

- **VON OLSON et al.** Higher-Alcohols Biorefinery - Improvement of Catalyst for Ethanol Conversion. *Appl. Biochem. Biotechnol.,* 2004, vol. 113 (116), 913-932